# EUROPEAN PATENT APPLICATION

(11) **EP 4 049 679 A1**
(43) Date of publication of application: **31.08.2022**
(21) Application number: 20879038.6
(22) Date of filing: 20.10.2020
(51) Int. Cl.: A61K 45/00, A61P 25/28, G01N 33/15, G01N 33/50, A61K 31/197

(54) **PREVENTATIVE OR THERAPEUTIC AGENT FOR TAUOPATHY**

(30) Priority: 25.10.2019 JP 2019194758
(71) Applicant: Kyoto University, Kyoto-shi, Kyoto 606-8501 (JP); National Institutes for Quantum Science and Technology, Chiba 263-8555 (JP)
(72) Inventor: INOUE, Haruhisa, Kyoto-shi, Kyoto 606-8501 (JP); IMAMURA, Keiko, Kyoto-shi, Kyoto 606-8501 (JP); HIGUCHI, Makoto, Chiba-shi, Chiba 263-8555 (JP); SAHARA, Naruhiko, Chiba-shi, Chiba 263-8555 (JP); ONO, Maiko, Chiba-shi, Chiba 263-8555 (JP)
(74) Representative: Heubeck, Christian
(86) International application number: PCT/JP2020/039450
(87) International publication number: WO 2021/079887

(57) **Abstract**

A prophylactic or therapeutic agent for tauopathy, containing an inhibitor of α₂δ of the voltage-dependent calcium channel is provided by the present invention.

## Description

### [Technical Field]

The present invention relates to a novel prophylactic or therapeutic agent for tauopathy. More specifically, the present invention relates to a prophylactic or therapeutic agent for tauopathy, containing an inhibitor of calcium ion influx into cells.

### [Background Art]

The number of patients with dementia is increasing with the aging of society, and most of them are estimated to be tauopathy patients. Tauopathy is a general term for neurodegenerative diseases that accompany aggregation and intracellular accumulation of tau protein in the brain, in which tau aggregation is considered to contribute to the onset of the disease. Representative disease includes Alzheimer's disease and frontotemporal lobar degeneration-Tau (FTLD-Tau) which shows tauopathy. Since no effective therapeutic method has been established for these, the development of a drug having a therapeutic or prophylactic effect is desired.

In recent years, due to the advances in induced pluripotent stem cell (iPS cell) technology, cell models for various neurodegenerative diseases have been created. The present inventors have previously reported that induced pluripotent stem cells can be rapidly and efficiently induced to differentiate into glutamatergic cerebral cortical neurons by forcibly expressing Neurogenin2 (Ngn2) in the cells (Patent Literature 1). Then, they have clarified that the cerebral cortical neurons produced from familial FTLD patient-derived iPS cells by using this method show spontaneous tau oligomerization that leads to cell death, that is, the cell is an excellent tauopathy cell model (Patent Literature 2).

Furthermore, the present inventors have clarified by analysis using hM4Di, which is an inhibitory designer receptor exclusively activated by designer drug (DREADD), that the suppression of neural activity reduces oligomerization of tau and cell death in the aforementioned tauopathy cell model (Patent Literature 3, Non Patent Literature 1). They have clarified that oligomerization of tau and cell death can also be reduced with NMDA type or AMPA type glutamate receptor antagonists (AP5 (APV, R-2-amino-5-phosphonopentanoate), CNQX (6-cyano-7-nitroquinoxaline-2,3-dione)) (Patent Literatures 2, 3, Non Patent Literature 1).

These findings suggest that a drug capable of inhibiting calcium influx associated with neural activity can be a prophylactic or therapeutic drug for tauopathy. However, it still remains unclear which of the proteins involved in calcium influx is particularly important for the pathology of tauopathy.

Incidentally, a new research concept called drug repositioning (DR) has been the subject of discussion as a method for breaking the deadlock in research and development of new drug that has been seen these days. It aims to discover new efficacy of existing drugs whose safety and pharmacokinetics in humans have already been confirmed by actual results, and to achieve practical application thereof. Since many existing data are available for use, further advantages are afforded such as suppressed developmental costs, the presence of accumulated know-how and materials (related compound and the like), and the like.

### [Citation List]

### [Patent Literature]

[PTL 1]
   WO 2014/148646
[PTL 2]
   WO 2016/076435
[PTL 3]
   WO 2018/066701

### [Non Patent Literature]

### [NPL 1]

Imamura, K., et al., Calcium dysregulation contributes to neurodegeneration in FTLD patient iPSC-derived neurons. Sci. Rep., 6:34904-34913, 2016.

### [Summary of Invention]

### [Technical Problem]

Therefore, the problem of the present invention is to provide a prophylactic or therapeutic means for tauopathy by drug repositioning which uses an existing drug whose safety and pharmacokinetics in humans have already been confirmed by actual results.

### [Solution to Problem]

The present inventors showed cell death suppressive effects of AP5 and CNQX on tauopathy model cells. Since the use of these drugs is limited to experimental ones not using humans, practicalization thereof as a preventive/therapeutic agent is expected to still require a considerable way to go. Therefore, the present inventors tried to search for a drug that affords the same effect from the existing drugs. Specifically, about 200 kinds of various existing drugs and 6 kinds of therapeutic agents for epilepsy were analyzed for the cell death suppressive effect on the aforementioned tauopathy cell model. As a result, it was surprisingly clarified that gabapentine, which is not a direct inhibitor of voltage-dependent calcium channel but merely a ligand for α₂δ, which is one of the auxiliary subunits thereof, shows a remarkable cell death suppressive effect. Furthermore, it was confirmed that gabapentine also has an effect of inhibiting the formation of tau oligomers.

It was also confirmed that pregabalin and mirogabalin, which are known as inhibitors of α₂δ like gabapentine, have a remarkable effect of inhibiting cell death and tau oligomer formation in the aforementioned tauopathy cell model.

It was further shown that gabapentine can also inhibit tau aggregation that occurs in the brain of a tauopathy mouse model. Based on these results, the present invention has been completed.

Accordingly, the present invention provides the following.
[1] A prophylactic or therapeutic agent for tauopathy, comprising an inhibitor of α₂δ of a voltage-dependent calcium channel.
[2] The agent of [1], wherein the aforementioned inhibitor of α₂δ is a compound represented by the formula (1): 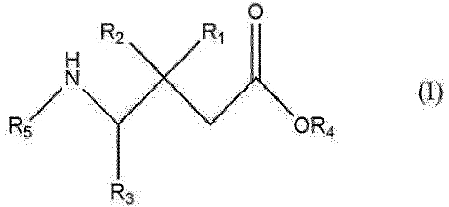 [in the formula (I),
   R₁ is a straight chain or branched alkyl group having 1 - 6 carbon atoms;
   R₂ is a hydrogen atom or a straight chain or branched alkyl group having 1 - 6 carbon atoms, or R₁ and R₂ are bonded to form a cycloalkane having 4 - 6 carbon atoms and optionally condensed with a 5-membered carbocycle, wherein the 5-membered carbocycle is optionally substituted by an alkyl group having 1 - 6 carbon atoms, an alkenyl group having 2 - 6 carbon atoms or a cycloalkyl group having 3 - 7 carbon atoms;
   R₃ is a hydrogen atom, a methyl group or a carboxyl group;
   R₄ is a hydrogen atom or a straight chain or branched alkyl group having 1 - 6 carbon atoms;
   R₅ is a hydrogen atom or a group represented by the formula (II): 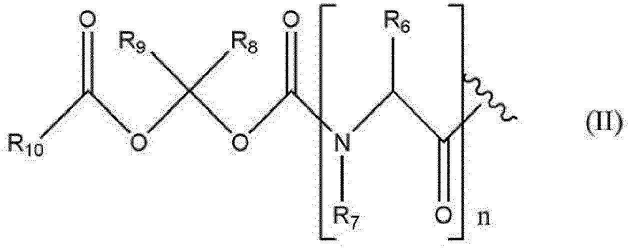 [in the formula (II),
   n is 0 or 1;
   R₆ is hydrogen, alkyl, substituted alkyl, alkoxy, substituted alkoxy, acyl, substituted acyl, alkoxycarbonyl, substituted alkoxycarbonyl, aryl, substituted aryl, arylalkyl, substituted arylalkyl, carbamoyl, substituted carbamoyl, cycloalkyl, substituted cycloalkyl, cycloheteroalkyl, substituted cycloheteroalkyl, heteroalkyl, substituted heteroalkyl, heteroaryl, substituted heteroaryl, heteroarylalkyl or substituted heteroarylalkyl, or R₆ and R₇ optionally form, together with an atom bonded thereto, cycloheteroalkyl or a substituted cycloheteroalkyl ring;
   R₇ is hydrogen, alkyl, substituted alkyl, aryl, substituted aryl, arylalkyl, substituted arylalkyl, cycloalkyl, substituted cycloalkyl, cycloheteroalkyl, substituted cycloheteroalkyl, heteroalkyl, substituted heteroalkyl, heteroaryl, substituted heteroaryl, heteroarylalkyl or substituted heteroarylalkyl;
   R₈ and R₉ are each independently hydrogen, alkyl, substituted alkyl, alkoxycarbonyl, substituted alkoxycarbonyl, aryl, substituted aryl, arylalkyl, substituted arylalkyl, carbamoyl, substituted carbamoyl, cycloalkyl, substituted cycloalkyl, heteroalkyl, substituted heteroalkyl, heteroaryl, substituted heteroaryl, heteroarylalkyl or substituted heteroarylalkyl, or R₈ and R₉ optionally form, together with an atom bonded thereto, cycloalkyl, substituted cycloalkyl, cycloheteroalkyl or a substituted cycloheteroalkyl ring; and
   R₁₀ is acyl, substituted acyl, alkyl, substituted alkyl, aryl, substituted aryl, arylalkyl, substituted arylalkyl, cycloalkyl, substituted cycloalkyl, cycloheteroalkyl, substituted cycloheteroalkyl, heteroalkyl, substituted heteroalkyl, heteroaryl, substituted heteroaryl, heteroarylalkyl or substituted heteroarylalkyl]],
   or a salt thereof.
[3] The agent of [2], wherein the aforementioned inhibitor of α₂δ is one or more compounds selected from gabapentine, pregabalin, mirogabalin and gabapentine enacarbil, and analogs thereof, or a salt thereof.
[4] The agent of [3], wherein the aforementioned inhibitor of α₂δ is one or more compounds selected from gabapentine and pregabalin, or a salt thereof.
[5] The agent of any of [1] to [4], wherein the agent is an inhibitor of misfolding of a tau protein.
[6] The agent of any of [1] to [4], wherein the aforementioned tauopathy is Alzheimer's disease or frontotemporal lobar degeneration showing tauopathy (FTLD-Tau).
[7] The agent of [6], wherein the tauopathy is caused by MAPT gene mutation.
[8] A method for screening for a prophylactic or therapeutic agent for tauopathy, comprising the following steps:
   (1) a step of contacting α₂δ of a voltage-dependent calcium channel with a test substance, and
   (2) a step of selecting a test substance that has bound to α₂δ in step (1) as a candidate for a prophylactic or therapeutic agent for tauopathy.
[9] A method for preventing or treating tauopathy in a mammal, comprising administering an effective amount of an inhibitor of α₂δ to the mammal.
[10] The method of [9], wherein the aforementioned inhibitor of α₂δ is a compound represented by the formula (1): 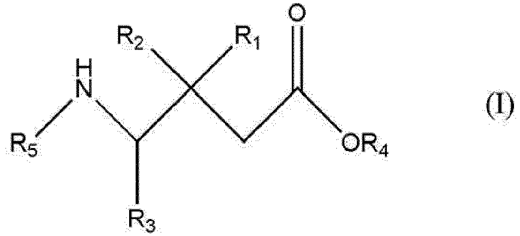 [in the formula (I),
   R₁ is a straight chain or branched alkyl group having 1 - 6 carbon atoms;
      R₂ is a hydrogen atom or a straight chain or branched alkyl group having 1 - 6 carbon atoms, or R₁ and R₂ are bonded to form a cycloalkane having 4 - 6 carbon atoms and optionally condensed with a 5-membered carbocycle, wherein the 5-membered carbocycle is optionally substituted by an alkyl group having 1 - 6 carbon atoms, an alkenyl group having 2 - 6 carbon atoms or a cycloalkyl group having 3 - 7 carbon atoms;
   R₃ is a hydrogen atom, a methyl group or a carboxyl group;
   R₄ is a hydrogen atom or a straight chain or branched alkyl group having 1 - 6 carbon atoms; and
   R₅ is a hydrogen atom or a group represented by the formula (II): 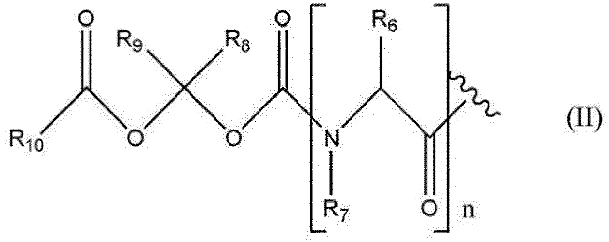 [in the formula (II),
   n is 0 or 1;
   R₆ is hydrogen, alkyl, substituted alkyl, alkoxy, substituted alkoxy, acyl, substituted acyl, alkoxycarbonyl, substituted alkoxycarbonyl, aryl, substituted aryl, arylalkyl, substituted arylalkyl, carbamoyl, substituted carbamoyl, cycloalkyl, substituted cycloalkyl, cycloheteroalkyl, substituted cycloheteroalkyl, heteroalkyl, substituted heteroalkyl, heteroaryl, substituted heteroaryl, heteroarylalkyl or substituted heteroarylalkyl, or R₆ and R₇ optionally form, together with an atom bonded thereto, cycloheteroalkyl or a substituted cycloheteroalkyl ring;
   R₇ is hydrogen, alkyl, substituted alkyl, aryl, substituted aryl, arylalkyl, substituted arylalkyl, cycloalkyl, substituted cycloalkyl, cycloheteroalkyl, substituted cycloheteroalkyl, heteroalkyl, substituted heteroalkyl, heteroaryl, substituted heteroaryl, heteroarylalkyl or substituted heteroarylalkyl;
   R₈ and R₉ are each independently hydrogen, alkyl, substituted alkyl, alkoxycarbonyl, substituted alkoxycarbonyl, aryl, substituted aryl, arylalkyl, substituted arylalkyl, carbamoyl, substituted carbamoyl, cycloalkyl, substituted cycloalkyl, heteroalkyl, substituted heteroalkyl, heteroaryl, substituted heteroaryl, heteroarylalkyl or substituted heteroarylalkyl, or R₈ and R₉ optionally form, together with an atom bonded thereto, cycloalkyl, substituted cycloalkyl, cycloheteroalkyl or a substituted cycloheteroalkyl ring; and
   R₁₀ is acyl, substituted acyl, alkyl, substituted alkyl, aryl, substituted aryl, arylalkyl, substituted arylalkyl, cycloalkyl, substituted cycloalkyl, cycloheteroalkyl, substituted cycloheteroalkyl, heteroalkyl, substituted heteroalkyl, heteroaryl, substituted heteroaryl, heteroarylalkyl or substituted heteroarylalkyl]],
   or a salt thereof.
[11] The method of [9] or [10], wherein the aforementioned inhibitor of α₂δ is one or more compounds selected from gabapentine, pregabalin, mirogabalin and gabapentine enacarbil, and analogs thereof, or a salt thereof.
[12] An inhibitor of α₂δ for use in the treatment or prophylaxis of tauopathy.
[13] The inhibitor of α₂δ of [12], wherein the inhibitor is a compound represented by the formula (1): 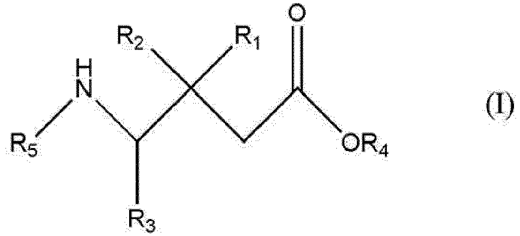 [in the formula (I),
   R₁ is a straight chain or branched alkyl group having 1 - 6 carbon atoms;
      R₂ is a hydrogen atom or a straight chain or branched alkyl group having 1 - 6 carbon atoms, or R₁ and R₂ are bonded to form a cycloalkane having 4 - 6 carbon atoms and optionally condensed with a 5-membered carbocycle, wherein the 5-membered carbocycle is optionally substituted by an alkyl group having 1 - 6 carbon atoms, an alkenyl group having 2 - 6 carbon atoms or a cycloalkyl group having 3 - 7 carbon atoms;
   R₃ is a hydrogen atom, a methyl group or a carboxyl group;
   R₄ is a hydrogen atom or a straight chain or branched alkyl group having 1 - 6 carbon atoms; and
   R₅ is a hydrogen atom or a group represented by the formula (II): 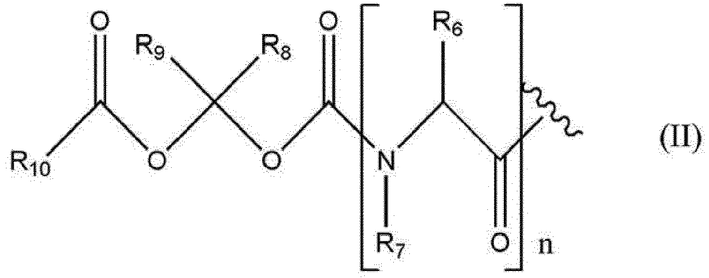 [in the formula (II),
   n is 0 or 1;
   R₆ is hydrogen, alkyl, substituted alkyl, alkoxy, substituted alkoxy, acyl, substituted acyl, alkoxycarbonyl, substituted alkoxycarbonyl, aryl, substituted aryl, arylalkyl, substituted arylalkyl, carbamoyl, substituted carbamoyl, cycloalkyl, substituted cycloalkyl, cycloheteroalkyl, substituted cycloheteroalkyl, heteroalkyl, substituted heteroalkyl, heteroaryl, substituted heteroaryl, heteroarylalkyl or substituted heteroarylalkyl, or R₆ and R₇ optionally form, together with an atom bonded thereto, cycloheteroalkyl or a substituted cycloheteroalkyl ring;
   R₇ is hydrogen, alkyl, substituted alkyl, aryl, substituted aryl, arylalkyl, substituted arylalkyl, cycloalkyl, substituted cycloalkyl, cycloheteroalkyl, substituted cycloheteroalkyl, heteroalkyl, substituted heteroalkyl, heteroaryl, substituted heteroaryl, heteroarylalkyl or substituted heteroarylalkyl;
   R₈ and R₉ are each independently hydrogen, alkyl, substituted alkyl, alkoxycarbonyl, substituted alkoxycarbonyl, aryl, substituted aryl, arylalkyl, substituted arylalkyl, carbamoyl, substituted carbamoyl, cycloalkyl, substituted cycloalkyl, heteroalkyl, substituted heteroalkyl, heteroaryl, substituted heteroaryl, heteroarylalkyl or substituted heteroarylalkyl, or R₈ and R₉ optionally form, together with an atom bonded thereto, cycloalkyl, substituted cycloalkyl, cycloheteroalkyl or a substituted cycloheteroalkyl ring; and
   R₁₀ is acyl, substituted acyl, alkyl, substituted alkyl, aryl, substituted aryl, arylalkyl, substituted arylalkyl, cycloalkyl, substituted cycloalkyl, cycloheteroalkyl, substituted cycloheteroalkyl, heteroalkyl, substituted heteroalkyl, heteroaryl, substituted heteroaryl, heteroarylalkyl or substituted heteroarylalkyl]],
   or a salt thereof.
[14] The inhibitor of α₂δ of [12] or [13], wherein the inhibitor is one or more compounds selected from gabapentine, pregabalin, mirogabalin and gabapentine enacarbil, and analogs thereof, or a salt thereof.
[15] Use of an inhibitor of α₂δ in the production of a prophylactic or therapeutic agent for tauopathy.

### [Advantageous Effects of Invention]

According to the present invention, the prophylaxis and/or treatment of tauopathy become(s) possible. In particular, one containing an existing drug with confirmed safety as an active ingredient has less concern of side effects.

### [Brief Description of Drawings]

[Fig. 1]

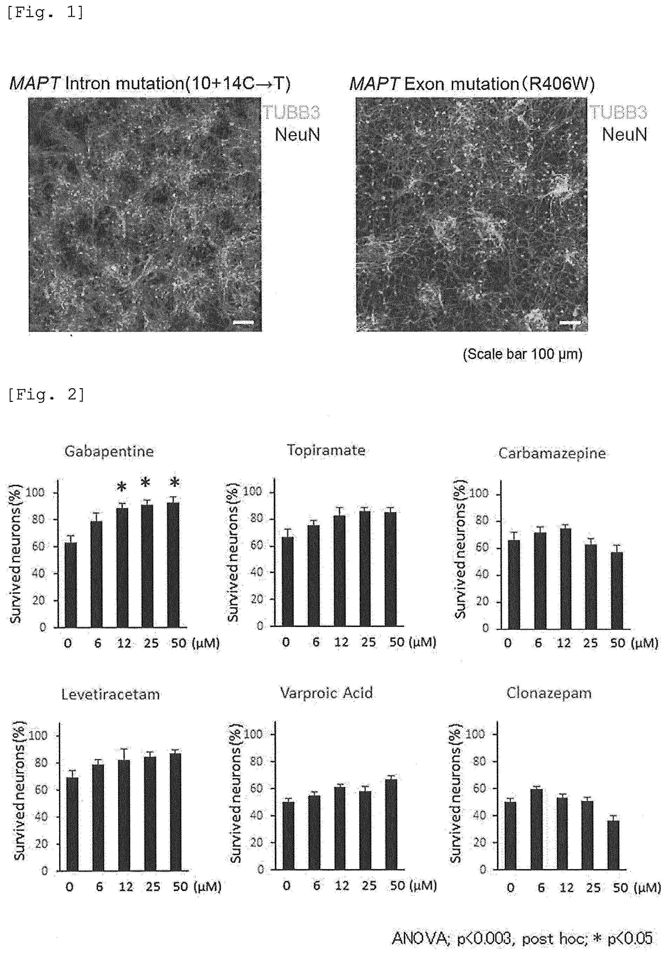

   Fig. 1 shows the results of double immunofluorescence staining with βIII tubulin (TUBB3) and NeuN of cerebral cortical neurons induced from iPS cells derived from FTLD-Tau patients with either an intron mutation (intron 10 + 14C→T) or an exon mutation (R406W) in the MAPT gene. scale bar=100 µm
[Fig. 2]
   Fig. 2 shows the analysis results of the suppressive effect of 6 kinds of existing drugs-which are well known as inhibitors of ion channels and ion exchange transporters-on the cell death of cerebral cortical neurons induced to differentiate from iPS cells derived from FTLD-Tau patients with an intron mutation (intron 10 + 14C→T). The horizontal axis shows the concentration of each compound, and the vertical axis shows the survival rate (= number of surviving cells on Day 21/number of surviving cells on Day 8)×100) of the aforementioned neurons (n = 3; one-way ANOVA, p < 0.05; post hoc test, *p < 0.05). The error bar shows standard error (SEM).
[Fig. 3]

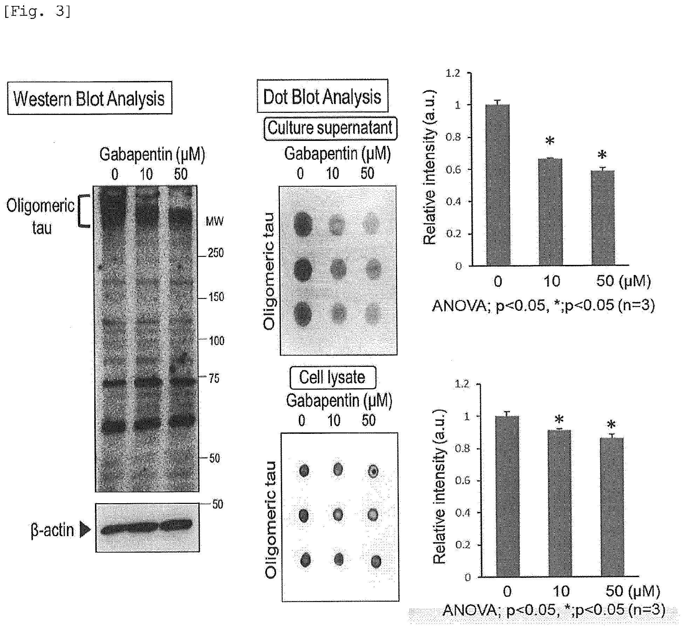

   Fig. 3 shows the analysis results of the suppressive effect of gabapentine on the misfolding of tau protein that occurs in cerebral cortical neurons induced to differentiate from iPS cells derived from FTLD-Tau patients with an exon mutation (R406W). The left figure of Fig. 3 shows the results of Western blot analysis, the central figure of Fig. 3 shows the results of dot blot analysis, and the right figure of Fig. 3 is a graph showing quantified results of the aforementioned dot blot analysis (n=3; one-way ANOVA, p < 0.05; post hoc test, * p < 0.05). The error bar shows standard error (SEM). In the central figure and the right figure of Fig. 3, the upper figure shows the results when the culture supernatant was used as the sample, and the lower figure shows the results when the cell lysate was used as the sample.
[Fig. 4]

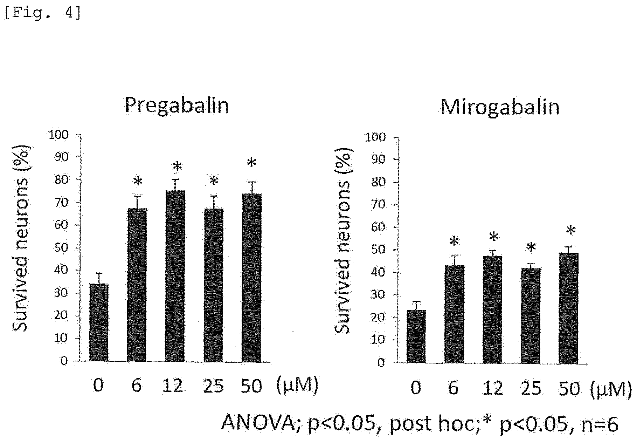

   Fig. 4 shows the analysis results of the suppressive effect of pregabalin and mirogabalin on the cell death of cerebral cortical neurons induced to differentiate from iPS cells derived from FTLD-Tau patients (n=6; one-way ANOVA, p < 0.05; post hoc test, * p < 0.05). The detail is the same as in Fig. 2.
[Fig. 5]

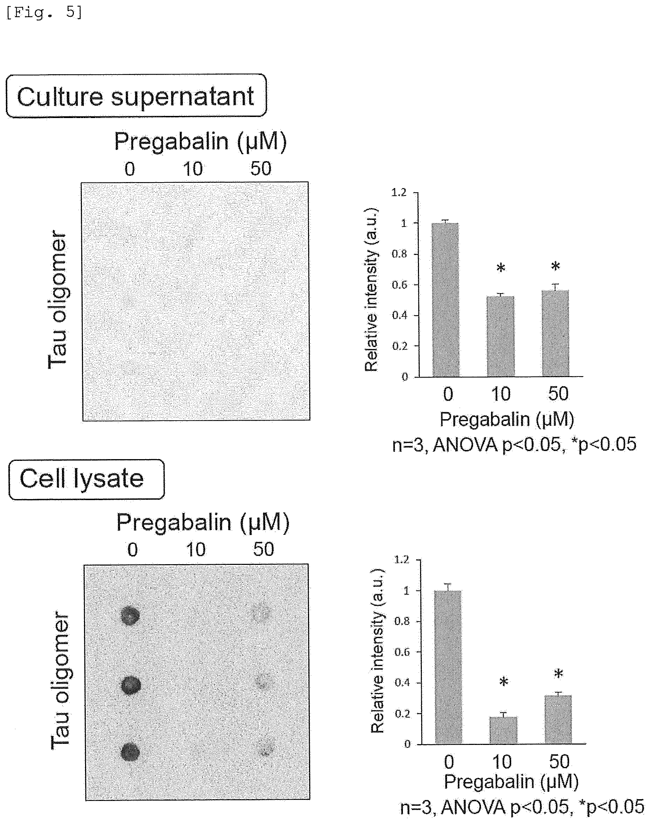

   Fig. 5 shows the analysis results (dot blot analysis) of the suppressive effect of pregabalin on the misfolding of tau protein that occurs in cerebral cortical neurons induced to differentiate from iPS cells derived from FTLD-Tau patients. The detail is the same as in Fig. 3.
[Fig. 6]

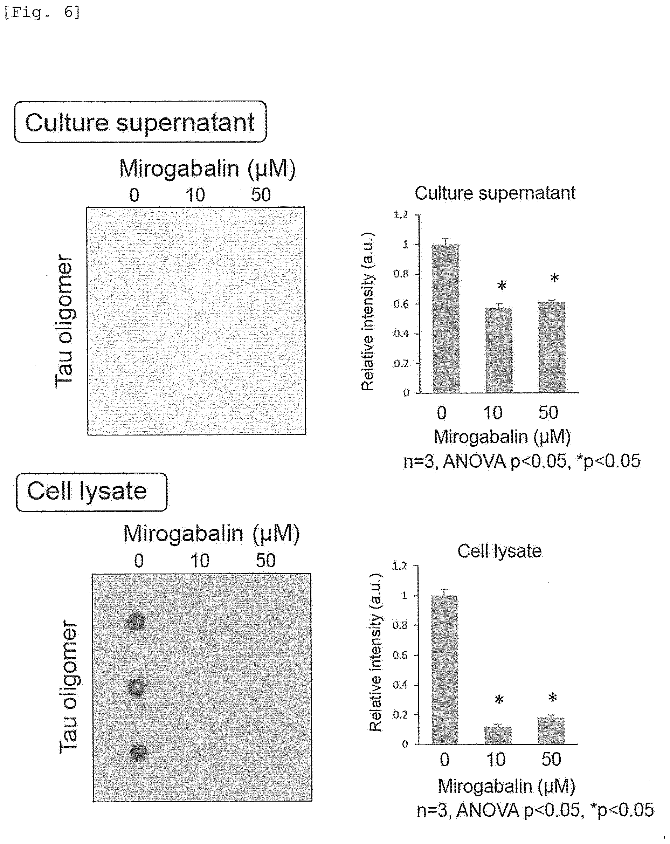

   Fig. 6 shows the analysis results (dot blot analysis) of the suppressive effect of mirogabalin on the misfolding of tau protein that occurs in cerebral cortical neurons induced to differentiate from iPS cells derived from FTLD-Tau patients. The detail is the same as in Fig. 3.
[Fig. 7]

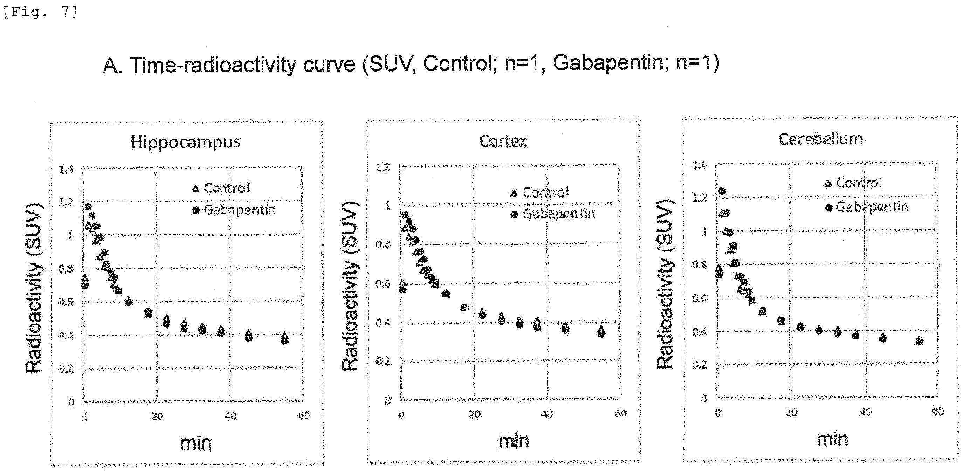

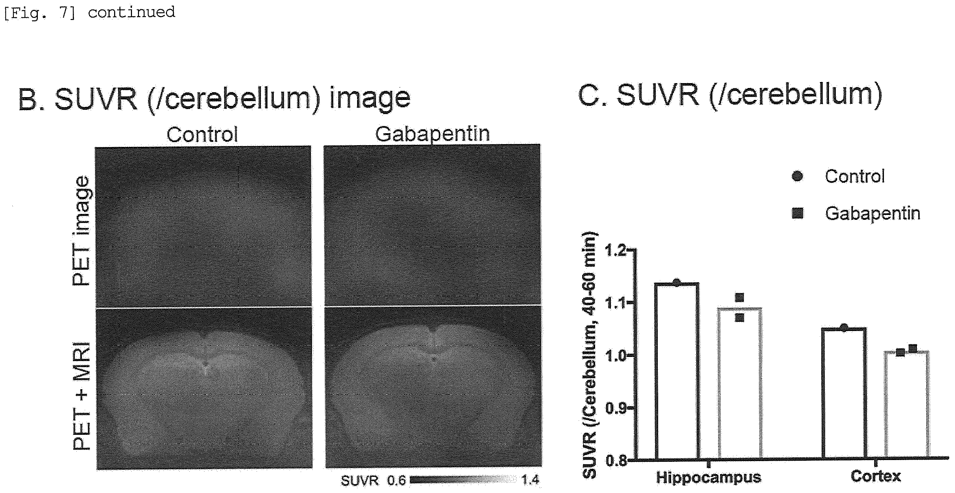

   Fig. 7 shows the analysis results, using Positron Emission Tomography (PET), of the suppressive effect of gabapentine (oral administration) on tau aggregation that occurs in the brain of tauopathy mouse model. Fig. 7A shows the time-radioactivity curve data for [¹⁸F]PM-PBB in gabapentin-administered and non-administered mice. Fig. 7B shows a Standardized uptake value ratio (SUVR) image (upper) with the cerebellum as the target region of the brain cross section including the hippocampus and cerebral cortex 40-60 min after intravenous injection of [¹⁸F]PM-PBB3, and a superimposed image (lower) of the aforementioned SUVR image and the MRI image. Fig. 7C is a graph showing the average value of radioactivity obtained in the SUVR image of Fig. 7B (gabapentin-administered mouse: n=2, non-administered mouse: n=1) .

### [Description of Embodiments]

### 1. The agent of the present invention

The present invention provides a prophylactic or therapeutic agent for tauopathy, containing an inhibitor of α₂δ of a voltage-dependent calcium channel (hereinafter the term "the agent of the present invention" is sometimes used as a term encompassing a prophylactic agent and a therapeutic agent). In another embodiment, the present invention provides an inhibitor of α₂δ for use in the treatment or prophylaxis of tauopathy. In still another embodiment, the present invention also provides use of an inhibitor of α₂δ in the production of a prophylactic or therapeutic agent for tauopathy.

### <<Tau>>

Tau is a microtubule-associated protein that is mainly expressed in the nervous system. It promotes the polymerization of tubulin and stabilizes microtubules, and contributes to the construction and maintenance of neural axons. It is encoded by the MAPT gene, and selective splicing thereof causes the expression of six kinds of isoforms in the human brain. In particular, selective splicing of exon 10 is important, and the splicing of the exons produces a 3R type (3 repeat tau) with 3 repeats involved in microtubule binding, and no splicing produces a 4R type (4 repeat tau) with four of the sequences. Generally, the expression level of the 4 repeat tau and the expression level of the 3 repeat tau are almost of the same level in the brain of a human adult.

It is known that when any isoform is excessively phosphorylated, it loses the ability to bind to microtubules and self-aggregates (Lee V.M., et al., Annu. Rev. Neurosci., 24:1121-159, 2001).

### <<Tauopathy>>

As described above, tauopathy is a generic term for neurodegenerative diseases that accompany aggregation and intracellular accumulation of tau protein in the brain, where aggregation of tau is considered to contribute to the onset thereof. Representative tauopathy includes Alzheimer's disease (AD), frontotemporal lobar degeneration tauopathy (FTLD-tau), frontotemporal dementia (FTD), Pick's disease, progressive supranuclear palsy (PSP), corticobasal degeneration (CBD), argyrophilic grain disease, dementia with neurofibrillary tangles, diffuse neurofibrillary tangles with calcification (DNTC), pantothenate kinase-associated neurodegeneration (PKAN), β-propeller protein-associated neurodegeneration (BPAN), neurodegeneration with brain iron accumulation (NBIA), Huntington's disease, and the like. Among these, in frontotemporal dementia with Parkinsonism linked to chromosome 17 (FTDP-17), which is one type of FTLD-tau, many MAPT gene mutations have been identified in the patient, and the relationship between tau abnormalities and onset has been strenuously analyzed. The mutation is roughly classified into a type that causes a mutant tau protein (an exon mutation) and a type that causes abnormality in exon 10 splicing (mainly an intron mutation), and it has been reported that tau protein is aggregated and accumulated not only by an exon mutation but also an intron mutation (Hutton, M., et al., Association of missense and 5'-splice-site mutations in tau with the inherited dementia FTDP-17. Nature 393, 702-705, 1998.).

An inhibitor of α₂δ, or the agent of the present invention, can be used as a prophylactic or therapeutic agent for any type of tauopathy mentioned above, preferably for Alzheimer's disease and FTLD-tau. The patients thereof may or may not have mutation of MAPT gene. In addition, the tauopathy may be familial or sporadic. Furthermore, when the MAPT gene has a mutation, it may be either a mutation of exon (e.g., mutation of R406W) or a mutation of intron (e.g., a mutation in intron 10 +14C→T in which cytosine, the 14th base of intron 10 of the MAPT gene, was replaced with thymine).

### «Voltage dependent calcium channel»

The voltage-dependent calcium channel (VDCC) is an ion channel that is activated and opened by sensing depolarization of the plasma membrane, and selectively allows permeation of Ca²⁺ from outside the cell to inside the cell. Depending on the opening potential, VDCC is roughly divided into L-type and non-L-type that are activated at high potential (up to -20 mV) and T-type that is activated at low potential (up to -60 mV), and the non-L-type is further divided into N type, P/Q type, and R type. VDCC is formed from a huge α₁ subunit that forms the channel itself (channel pore) and two or three kinds of auxiliary subunits (α₂δ, β, γ). Of these, the α₁ subunit determines the characteristics of the channel, and the auxiliary subunits are considered to contribute to the regulation of expression and intracellular localization of the α₁ subunit (Bauer CS., et al., A new look at calcium channel α2δ subunits. Curr Opin Neurobiol., 20:563-71, 2010.).

The α₂δ subunit (sometimes to be abbreviated as "α₂δ" in the present specification) is a dimer in which α₂ and δ encoded by a single gene are linked by a disulfide bond, and four types of isoforms are known (α₂δ-1, α₂δ-2, α₂δ-3, α₂δ-4). The gene encoding α₂δ-1 is known as CACNA2D1 (GeneBank Accession No: NM_000722 (human)), the gene encoding α₂δ-2 is known as CACNA2D2 (GeneBank Accession No: NM_001005505 (human)), the gene encoding α₂δ-3 is known as CACNA2D3 (GeneBank Accession No: NM_018398 (human)), and the gene encoding α₂δ-4 is known as CACNA2D4 (GeneBank Accession No: NM_172364 (human)). α₂δ is expressed in a form-in which α₂ and δ are fused (Pro-form)-from the gene encoding α₂δ, and then is divided into α₂ and δ by posttranslational modification, and a disulfide bond is formed between α₂ and δ to give a dimer (Dolphin A.C., Biochim BiophysActa., 1828(7):1541-9 (2013)). The α₂δ subunit is known to contribute to the transport of the α₁ subunit to the plasma membrane (Davies A., et al., Trends Pharmacol Sci., 28(5):220-8 (2007)). While the α₂δ isoform targeted by the inhibitor of α₂δ used in the present invention is not particularly limited, α₂δ-1, α₂δ-2 and α₂δ-3 which are highly expressed in the central nervous system are preferred, and α₂δ-1 and α₂δ-2 are particularly preferred.

### <<Inhibitor of α₂δ>>

In the present specification, the "inhibitor of α₂δ" (to be also referred to as "the compound of the present invention") means a compound that inhibits the functions of α₂δ (e.g., induction of expression of α₁ subunit, transport of α₁ subunit to plasma membrane, etc.). The inhibition can indirectly suppress the intracellular influx of calcium ion by VDCC. Therefore, in the present specification, the inhibitor of α₂δ does not include a substance that binds to the VDCC channel itself to directly inhibit calcium influx by VDCC. Such inhibitor of α₂δ includes α₂δ ligand, antibody to α₂δ, aptamer, inhibitor of α₂δ expression, and the like.

In the present specification, the "α₂δ ligand" means a compound that can bind to α₂δ to inhibit the functions of α₂δ, and the α₂δ ligand used in the present invention is not particularly limited as long as it can inhibit the functions of α₂δ. From the aspect of suppressing the development cost low, existing drugs whose safety and pharmacokinetics in human have already been confirmed by actual results are preferred. From the aspect of reducing the side effects, moreover, compounds that specifically bind to α₂δ are preferred. Specifically, for example, a compound represented by the formula (1): 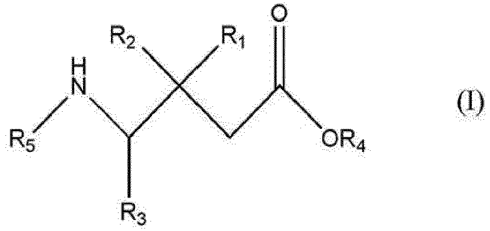 [in the formula (I),
R₁ is a straight chain or branched alkyl group having 1 - 6 carbon atoms;
   R₂ is a hydrogen atom or a straight chain or branched alkyl group having 1 - 6 carbon atoms, or R₁ and R₂ are bonded to form a cycloalkane having 4 - 6 carbon atoms and optionally condensed with a 5-membered carbocycle, wherein the 5-membered carbocycle is optionally substituted by an alkyl group having 1 - 6 carbon atoms, an alkenyl group having 2 - 6 carbon atoms or a cycloalkyl group having 3 - 7 carbon atoms;
R₃ is a hydrogen atom, a methyl group or a carboxyl group;
R₄ is a hydrogen atom or a straight chain or branched alkyl group having 1 - 6 carbon atoms; and
R₅ is a hydrogen atom or a group represented by the formula (II):

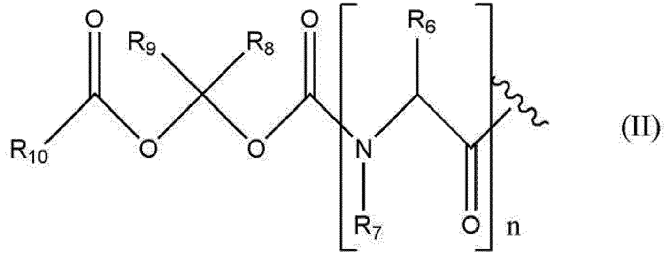

   [in the formula (II),
   n is 0 or 1;
   R₆ is hydrogen, alkyl, substituted alkyl, alkoxy, substituted alkoxy, acyl, substituted acyl, alkoxycarbonyl, substituted alkoxycarbonyl, aryl, substituted aryl, arylalkyl, substituted arylalkyl, carbamoyl, substituted carbamoyl, cycloalkyl, substituted cycloalkyl, cycloheteroalkyl, substituted cycloheteroalkyl, heteroalkyl, substituted heteroalkyl, heteroaryl, substituted heteroaryl, heteroarylalkyl or substituted heteroarylalkyl, or R₆ and R₇ optionally form, together with an atom bonded thereto, cycloheteroalkyl or a substituted cycloheteroalkyl ring;
   R₇ is hydrogen, alkyl, substituted alkyl, aryl, substituted aryl, arylalkyl, substituted arylalkyl, cycloalkyl, substituted cycloalkyl, cycloheteroalkyl, substituted cycloheteroalkyl, heteroalkyl, substituted heteroalkyl, heteroaryl, substituted heteroaryl, heteroarylalkyl or substituted heteroarylalkyl;
   R₈ and R₉ are each independently hydrogen, alkyl, substituted alkyl, alkoxycarbonyl, substituted alkoxycarbonyl, aryl, substituted aryl, arylalkyl, substituted arylalkyl, carbamoyl, substituted carbamoyl, cycloalkyl, substituted cycloalkyl, heteroalkyl, substituted heteroalkyl, heteroaryl, substituted heteroaryl, heteroarylalkyl or substituted heteroarylalkyl, or R₈ and R₉ optionally form, together with an atom bonded thereto, cycloalkyl, substituted cycloalkyl, cycloheteroalkyl or a substituted cycloheteroalkyl ring; and
   R₁₀ is acyl, substituted acyl, alkyl, substituted alkyl, aryl, substituted aryl, arylalkyl, substituted arylalkyl, cycloalkyl, substituted cycloalkyl, cycloheteroalkyl, substituted cycloheteroalkyl, heteroalkyl, substituted heteroalkyl, heteroaryl, substituted heteroaryl, heteroarylalkyl or substituted heteroarylalkyl]]
   can be mentioned.

A compound in which R₅ in the formula (I) is a group represented by the formula (II) functions as a prodrug of a compound wherein R₅ is a hydrogen atom. Further specific examples of the compound that functions as a prodrug include (1) a compound wherein R₈ is hydrogen, Rg is hydrogen, methyl, ethyl, propyl, isopropyl, n-butyl, isobutyl, sec-butyl, tert-butyl, cyclohexyl or phenyl, and R₁₀ is methyl, ethyl, propyl, isopropyl, n-butyl, isobutyl, sec-butyl, tert-butyl, pentyl, isopentyl, sec-pentyl, neopentyl, 1,1-dimethoxyethyl, 1,1-diethoxyethyl, cyclobutyl, cyclopentyl, cyclohexyl, phenyl, 4-methoxyphenyl, benzyl, phenethyl, styryl or 3-pyridyl, (2) a compound wherein R₈ and R₉ are each methyl, and R₁₀ is methyl, ethyl, propyl, isopropyl, butyl, isobutyl, sec-butyl, tert-butyl, pentyl, isopentyl, sec-pentyl, neopentyl, 1,1-dimethoxyethyl, 1,1-diethoxyethyl, cyclobutyl, cyclopentyl, cyclohexyl, phenyl, 4-methoxyphenyl, benzyl, phenethyl, styryl or 3-pyridyl, (3) a compound wherein R₈ is methyl, Rg is methoxycarbonyl, ethoxycarbonyl, isopropoxycarbonyl or cyclohexyloxycarbonyl, and R₁₀ is methyl, ethyl, propyl, isopropyl, butyl, isobutyl, sec-butyl, tert-butyl, pentyl, isopentyl, sec-pentyl, neopentyl, 1,1-dimethoxyethyl, 1,1-diethoxyethyl, cyclobutyl, cyclopentyl, cyclohexyl, phenyl, 4-methoxyphenyl, benzyl, phenethyl, styryl or 3-pyridyl, (4) a compound wherein R₉ and R₈ form a cyclohexyl ring with the atom bonded thereto, and R₁₀ is methyl, ethyl, propyl, isopropyl, butyl, isobutyl, sec-butyl, tert-butyl, pentyl, isopentyl, sec-pentyl, neopentyl, 1,1-dimethoxyethyl, 1,1-diethoxy ethyl, cyclobutyl, cyclopentyl, cyclohexyl, phenyl, 4-methoxyphenyl, benzyl, phenethyl, styryl or 3-pyridyl, (5) a compound wherein R₈ is hydrogen, Rg is hydrogen, methyl, ethyl, n-propyl, isopropyl, n-butyl, isobutyl, sec-butyl or tert-butyl, and R₁₀ is methyl, ethyl, n-propyl, isopropyl, n-butyl, isobutyl, sec-butyl, tert-butyl, 1,1-dimethoxyethyl, 1,1-diethoxyethyl, cyclobutyl, cyclopentyl or cyclohexyl, (6) a compound wherein R₈ is hydrogen, Rg is methyl, n-propyl or isopropyl, and R₁₀ is ethyl or isopropyl, (7) a compound wherein R₈ is hydrogen, Rg is methyl, and R₁₀ is isopropyl, (8) a compound wherein R₈ is hydrogen, Rg is methyl, and R₁₀ is ethyl or isopropyl, (9) a compound wherein R₈ is hydrogen, Rg is methyl, and R₁₀ is isopropyl, and the like. Further specifically, a compound represented by the formula (I) is, for example, gabapentine enacarbil.

The terms described in the explanation of each group of the above-mentioned formula (I) follow the definition and explanation in Japanese Translation of PCT Application Publication No. 2005-529941. The above-mentioned compound represented by the formula (1) can be produced by a method known per se and, for example, the method described in Japanese Translation of PCT Application Publication No. 2005-529941, and the like can be mentioned.

In the present specification, the "substituted" (to be also referred to simply as "substitution") means that one or more hydrogen atoms are replaced with a substituent. Typical substituents include, but are not limited to, -M, -R⁶⁰, -O-, =O, -OR⁶⁰, -SR⁶⁰, -S-, =S, -NR⁶⁰R⁶¹, =NR⁶⁰, -CF₃, -CN, -OCN, -SCN, -NO, -NO₂, =N₂, -N₃, -S(O)₂O-, -S(O)₂OH, -S(O)₂R⁶⁰, -OS(O₂)O-, - OS(O)₂R⁶⁰, -P(O)(O⁻)₂, -P(O)(OR⁶⁰)(O⁻), -OP(O)(OR⁶⁰)(OR⁶¹), - C(O)R⁶⁰, -C(S)R⁶⁰, -C(O)OR⁶⁰, -C(O)NR⁶⁰R⁶¹, -C(O)O⁻, -C(S)OR⁶⁰, - NR⁶²C(O)NR⁶⁰R⁶¹, -NR⁶²C(S)NR⁶⁰R⁶¹, -NR⁶²C(NR⁶³)NR⁶⁰R⁶¹, -C(NR⁶²)NR⁶⁰R⁶¹ (wherein M is independently halogen; R⁶⁰, R⁶¹, R⁶² and R⁶³ are each independently hydrogen, alkyl, substituted alkyl, alkoxy, substituted alkoxy, cycloalkyl, substituted cycloalkyl, cycloheteroalkyl, substituted cycloheteroalkyl, aryl, substituted aryl, heteroaryl or substituted heteroaryl, or, R⁶⁰ and R⁶¹ optionally form cycloheteroalkyl or a substituted cycloheteroalkyl ring together with the nitrogen atom bonded thereto; R⁶⁴ and R⁶⁵ are each independently hydrogen, alkyl, substituted alkyl, aryl, cycloalkyl, substituted cycloalkyl, cycloheteroalkyl, substituted cycloheteroalkyl, aryl, substituted aryl, heteroaryl or substituted heteroaryl, or R⁶⁴ and R⁶⁵ optionally form cycloheteroalkyl or a substituted cycloheteroalkyl ring together with the nitrogen atom bonded thereto). Preferably, the substituent includes -M, -R⁶⁰(=O)-OR⁶⁰, -SR⁶⁰, -S⁻, =S, -NR⁶⁰R⁶¹(=NR⁶⁰)-CF₃(-CN) -OCN- (-SCN) -NO, -NO₂, =N₂, -N₃, -S(O)₂R⁶⁰, -OS(O₂)O⁻, -OS(O)₂R⁶⁰, -P(O)(O⁻)₂, - P(O)(OR⁶⁰)(O⁻), -OP(O)(OR⁶⁰)(OR⁶¹), -C(O)R⁶⁰, -C(S)R⁶⁰, -C(O)OR⁶⁰, -C(O)NR⁶⁰R⁶¹, -C(O)NR⁶⁰R⁶¹, -C(O)O⁻, -NR⁶²C(O)NR⁶⁰R⁶¹, further preferably -M, -R⁶⁰, =O, -OR⁶⁰, -SR⁶⁰, -NR⁶⁰R⁶¹, -CF₃, -CN, -NO₂, - S(O)₂R⁶⁰, -P(O)(OR⁶⁰)(O⁻), -OP(O)(OR⁶⁰)(OR⁶¹), -C(O)R⁶⁰, -C(O)OR⁶⁰, -C(O)NR⁶⁰R⁶¹, -C(O)O⁻, most preferably -M, -R⁶⁰, =O, -OR⁶⁰, -SR⁶⁰, -NR⁶⁰R⁶¹, -CF₃, -CN, -NO₂, -S(O)₂R⁶⁰, -OP(O)(OR⁶⁰)(OR⁶¹), -C(O)R⁶⁰, -C(O)OR⁶⁰, and -C(O)O⁻ (wherein R⁶⁰, R⁶¹, and R⁶² are as defined above).

In the present specification, when R₆ - R₁₀, R⁶⁰ - R⁶⁵ contain a carbon atom, the number of respective carbon atoms is typically 1 - 20, preferably 1 - 10, further preferably 1 - 6.

Examples of the compound of the formula (1) wherein R₅ is a hydrogen atom include gabapentine, pregabalin, mirogabalin, analogs thereof, and the like.

As an analog of gabapentine, a compound of the above-mentioned formula (I), wherein R₁ and R₂ are bonded to form cycloalkane having 4 - 6 carbon atoms, preferably cycloalkane having 6 carbon atoms, R₃ is a hydrogen atom, a methyl group or a carboxyl group, preferably a hydrogen atom, R₄ is a hydrogen atom or a straight chain or branched alkyl group having 1 - 6 carbon atoms, preferably a hydrogen atom can be mentioned. A compound of the above-mentioned formula (I), wherein R₁ and R₂ are bonded to form cycloalkane having 6 carbon atoms, and R₃ and R₄ are each a hydrogen atom is gabapentine. Gabapentine and analogs thereof can be produced by a method known per se. For example, the method described in JP-A-51-88940, and the like can be mentioned.

In a pregabalin analog of the above-mentioned formula (I), R₁ is a straight chain or branched alkyl group having 1 - 6 carbon atoms, preferably an isobutyl group, R₂ is a hydrogen atom or a straight chain or branched alkyl group having 1 - 6 carbon atoms, preferably a hydrogen atom, R₃ is a hydrogen atom, a methyl group or a carboxyl group, preferably a hydrogen atom, and R₄ is a hydrogen atom or a straight chain or branched alkyl group having 1 - 6 carbon atoms, preferably a hydrogen atom. A compound of the above-mentioned formula (I) wherein R₁ is an isobutyl group, and R₂ - R₄ are each a hydrogen atom is pregabalin. Pregabalin and analogs thereof can be produced by a method known per se. For example, the method described in JP-A-2000-34226, and the like can be mentioned.

The analog of mirogabalin includes a compound represented by the following formula (III): 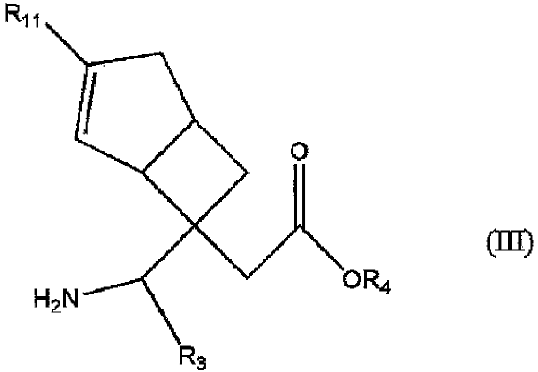 [wherein R₃ is a hydrogen atom, a methyl group or a carboxyl group, preferably a hydrogen atom;
R₄ is a hydrogen atom or a straight chain or branched alkyl group having 1 - 6 carbon atoms, preferably a hydrogen atom, and
R₁₁ is an alkyl group having 1 - 6 carbon atoms, an alkenyl group having 2 - 6 carbon atoms or a cycloalkyl group having 3 - 7 carbon atoms, preferably an ethyl group]. A compound of the above-mentioned formula (I) wherein R₃ is a hydrogen atom, R₄ is a hydrogen atom, and R₁₁ is an ethyl group is mirogabalin. Mirogabalin and analogs thereof can be produced by a method known per se. For example, the method described in WO2009/041453, and the like can be mentioned.

The compound of the present invention encompasses not only a free form, but also a pharmacologically acceptable salt thereof. The pharmacologically acceptable salt varies depending on the kind of the compound and, for example, base addition salts including salts with inorganic base such as alkali metal salt (sodium salt, potassium salt, etc.), alkaline earth metal salt (calcium salt, magnesium salt, etc.), aluminum salt, ammonium salt, and the like, and salts with organic base such as trimethylamine, triethylamine, pyridine, picoline, ethanolamine, diethanolamine, triethanolamine, dicyclohexylamine, N,N'-dibenzylethylenediamine, and the like, and the like, as well as acid addition salts including inorganic acid salts such as hydrochloride, hydrobromide, sulfate, hydroiodide, nitrate, phosphate and the like, and organic acid salts such as citrate, oxalate, acetate, formate, propionate, benzoate, trifluoroacetate, maleate, tartrate, methanesulfonate, benzenesulfonate, para-toluenesulfonate and the like, can be mentioned.

When the compound of the present invention has isomers such as optical isomer, stereoisomer, positional isomer, rotational isomer and the like, and any isomers and mixtures thereof are encompassed in the compound of the present invention. For example, when the compound of the present invention has an optical isomer, an optical isomer separated from a racemate is also encompassed in the compound of the present invention.

These isomers can be obtained as independent products by a synthesis means or a separation means (concentration, solvent extraction, column chromatography, recrystallization and the like) known *per se,* optical resolution methods (e.g., fractional recrystallization, chiral column method, diastereomer method, etc.) and the like.

The compound of the present invention may be a crystal, and both a single crystal and crystal mixtures are encompassed in the compound of the present invention. Crystals can be produced by crystallization according to crystallization methods known *per se.*

The compound of the present invention may be a solvate (e.g., hydrate etc.) or a non-solvate, both of which are encompassed in the compound of the present invention.

In addition, a compound labeled with an isotope (e.g., ³H, ¹⁴C, ³⁵S, ¹²⁵I and the like) is also encompassed in the compound of the present invention.

The anti-α₂δ antibody to be used in the present invention can be obtained as a polyclonal or monoclonal antibody by using a known means. Alternatively, a commercially available product may be used. While the origin of the antibody to be used in the present invention is not particularly limited, it is preferably a mammal-derived antibody, more preferably a human-derived antibody. A monoclonal antibody derived from a mammal may be either one produced by a hybridoma or one produced by a host transformed with an expression vector containing an antibody gene by a genetic engineering method. An antibody-producing hybridoma can be produced by a method known per se. For example, it can be produced by immunizing α₂δ or a part thereof used as an antigen according to a general immunization method, fusing the obtained immune cells with a known parent cell by a general cell fusion method, and screening for a monoclonal antibody-producing cell by a general screening method.

The aptamer to be used in the present invention may be a nucleic acid aptamer or a peptide aptamer. When it is a nucleic acid aptamer, the nucleic acid may be DNA, RNA, or a DNA/RNA chimera. In addition, it may be a ribose, a phosphate skeleton, a nucleobase, an amino acid residue, or a nucleic acid or peptide in which both terminal portions are modified. The nucleic acid aptamer may be double-stranded or single-stranded, preferably single-stranded. The aptamer in the present invention can be selected by a method well known to those skilled in the art. Without limitation, it can be selected by, for example, the SELEX method (Systematic Evolution of Ligands by Exponential Enrichment) (Tuerk, C. and Gold, L., 1990, Science, 249: 505-510) or the yeast Two-hybrid method.

The α₂δ expression inhibitor to be used in the present invention may act at any stage such as the transcription level, posttranscriptional regulation level, protein translation level, and posttranslational modification level, of the gene encoding α₂δ. Therefore, an expression inhibitor of α₂δ includes, for example, a nucleic acid that inhibits transcription of a gene encoding α₂δ (e.g., antigene), a nucleic acid that inhibits processing from an initial transcription product to mRNA, and a nucleic acid that inhibits translation from mRNA to protein (e.g., antisense nucleic acid, miRNA) or degrades mRNA (e.g., siRNA, ribozyme, microRNA (miRNA)).

The siRNA can be designed based on the cDNA sequence information of the α₂δ gene, and according to, for example, the rules proposed by Elbashir et al. (Genes Dev., 15, 188-200 (2001)). In addition, short hairpin RNA (shRNA), which is a precursor of siRNA, can be designed by appropriately selecting any linker sequence (e.g., about 5-25 bases) capable of forming a loop structure, and linking the sense strand and antisense strand of siRNA via the linker sequence.

The siRNA and/or shRNA sequences can be searched using search software provided free of charge on various websites. Examples of such site include, but are not limited to, the siDESIGN Center provided by Dharmacon (http://dharmacon.horizondiscovery.com/jp/design-center/?rdr=true&LangType=1041&pageid=17179928204), siRNA Target Finder provided by GenScript (https://www.genscript.com/tools/sirna-target-finder), and the like.

The miRNA can be searched using target prediction software provided free of charge on various websites. Examples of such site include, but are not limited to, TargetScan published by the Whitehead Institute in the United States (http://www.targetscan.org/vert_72/), DIANA-micro-T-CDS published by the Alexander Fleming Center for Biomedical Sciences, Greece (http://diana.imis.athena-innovation.gr/DianaTools/index.php?r=microT_CDS/index), and the like. Alternatively, miRNA that targets the mRNA encoding α₂δ can be searched using TarBase published by the Pasteur Institute of the University of Thessaly (http://carolina.imis.athena-innovation.gr/diana_tools/web/index.php?r=tarbasev8/index), which is a database relating to miRNAs that have been experimentally proven to act on target mRNAs, and the like.

The siRNA can be prepared by respectively synthesizing the sense strand and antisense strand of the target sequence on mRNA by a DNA/RNA automatic synthesizer, and denaturing them in an appropriate annealing buffer at about 90 - about 95°C for about 1 min, followed by annealing at about 30 - about 70°C for about 1 - about 8 hr. It can also be prepared by synthesizing shRNA to be a precursor of siRNA and cleaving same with a dicer. The miRNA and pre-miRNA can be synthesized by a DNA/RNA automatic synthesizer based on the sequence information thereof.

The antisense nucleic acid may be DNA, RNA, or a DNA/RNA chimera. When the antisense nucleic acid is a DNA, an RNA:DNA hybrid formed by the target RNA and the antisense DNA can be recognized by the endogenous RNase H and cause selective degradation of the target RNA. The length of the target region of the antisense nucleic acid is not particularly limited as long as the translation into protein is inhibited as a result of the hybridization of the antisense nucleic acid. The entire sequence or partial sequence of the mRNA encoding the protein may be used, and the short one may be about 10 bases, and the long one may be the full sequence of mRNA or initial transcription product. In addition, antisense nucleic acid may be one that not only hybridizes with target mRNA and initial transcription product to inhibit translation into protein, but also binds to these genes, which are double-stranded DNAs, to form triplex to inhibit transcription into RNA (antigenes).

The antisense nucleic acid can be prepared by determining the target sequence of mRNA or initial transcription product based on the cDNA sequence or genomic DNA sequence of the target gene, and synthesizing a sequence complementary thereto by a commercially available DNA/RNA automatic synthesizer.

As the agent of the present invention, the compound of the present invention as the active ingredient can be administered orally or parenterally as it is or after mixing the active ingredient with a pharmacologically acceptable carrier, excipient, diluent, etc. and forming a pharmaceutical composition in a suitable dosage form.

As the composition for oral administration, solid or liquid dosage form, specifically tablet (including sugar-coated tablet, film-coated tablet), pill, granule, powder, capsule (including soft capsule), syrup, emulsion, suspension and the like can be mentioned. On the other hand, as the composition for parenteral administration, injection, suppository and the like are used, and the injection may include dosage forms such as intravenous injection, subcutaneous injection, intracutaneous injection, intramuscular injection, instillation and the like. These preparations are produced by a well-known method using additives such as excipients (e.g., organic excipients including sugar derivatives such as lactose, sucrose, glucose, mannitol, sorbitol; starch derivatives such as cornstarch, potato starch, α starch, dextrin; cellulose derivatives such as crystalline cellulose; gum arabic; dextran; pullulan; and inorganic excipients including silicate derivatives such as light anhydrous silicic acid, synthetic aluminum silicate, calcium silicate, magnesium aluminometasilicate; phosphates such as calcium hydrogen phosphate; carbonates such as calcium carbonate; sulfates such as calcium sulfate, and the like), lubricants (e.g., stearic acid, stearic acid metal salts such as calcium stearate, magnesium stearate; talc; colloidal silica; waxes such as bead wax, cetaceum; boric acid; adipic acid; sulfates such as sodium sulfate; glycol; fumaric acid; sodium benzoate; DL leucine; lauryl sulfuric acid salts such as sodium lauryl sulfate, magnesium lauryl sulfate; silicic acids such as anhydrous silicic acid, silicic acid hydrate; and the above-mentioned starch derivatives), binders (e.g., hydroxypropylcellulose, hydroxypropylmethylcellulose, polyvinylpyrrolidone, macrogol, and compounds similar to the aforementioned excipients), disintegrants (e.g., cellulose derivatives such as low-substituted hydroxypropylcellulose, carboxymethylcellulose, carboxymethylcellulose calcium, internally-crosslinked carboxymethylcellulose sodium; chemically-modified starches and celluloses such as carboxymethyl starch, sodium carboxymethyl starch, crosslinked polyvinylpyrrolidone), emulsifier (e.g., colloidal clays such as bentonite, Veegum; metal hydroxides such as magnesium hydroxide, aluminum hydroxide; anionic surfactants such as sodium lauryl sulfate, calcium stearate; cationic surfactants such as benzalkonium chloride; and nonionic surfactants such as polyoxyethylene alkyl ether, polyoxyethylene sorbitan fatty acid ester, sucrose fatty acid ester), stabilizers (p-hydroxybenzoic acid esters such as methylparaben, propylparaben; alcohols such as chlorobutanol, benzyl alcohol, phenylethyl alcohol; benzalkonium chloride; phenols such as phenol, cresol; thimerosal; dehydroacetic acid; and sorbic acid), flavoring agents (e.g., sweetener, acidulant, flavor, and the like used generally), diluent and the like.

The dose of the compound of the present invention, which is the active ingredient of the agent of the present invention, may vary depending on various conditions such as the kind of the compound, the symptom, age, body weight, and drug acceptability of the administration subject, and the like. In the case of oral administration, the lower limit of 0.1 mg (preferably 0.5 mg) and the upper limit of 1000 mg (preferably 500 mg) per dose, and in the case of parenteral administration, the lower limit of 0.01 mg (preferably 0.05 mg) and the upper limit of 100 mg (preferably 50 mg) per dose can be administered to an adult 1 to 6 times per day. The dose may be increased or decreased depending on the symptoms. In particular, when the compound of the present invention has already been placed on the market as a pharmaceutical product for diseases other than the above-mentioned diseases, the dose of each compound can be appropriately selected from the range where the safety has been confirmed.

Furthermore, the agent of the present invention may be used in combination with other medicaments, for example, selective serotonin reuptake inhibitors (e.g., fluvoxamine, paroxetine, sertraline, etc.), serotonin 2A antagonists/reuptake inhibitors (e.g., trazodone, etc.) and the like. The agent of the present invention and these other medicaments can be administered simultaneously, successively, or separately.

A therapeutic and/or prophylactic method including administering an effective amount of the compound of the present invention or the agent of the present invention to a mammal (animal to be the subject of treatment or prophylaxis) is also included in the present invention. Examples of the animal include mouse, rat, hamster, rabbit, cat, dog, bovine, sheep, monkey, and human, and human is preferred.

### 2. The method of the present invention

The present invention also provides a method for screening for a prophylactic or therapeutic agent for tauopathy, including the following steps:
(1) a step of contacting α₂δ of a voltage-dependent calcium channel with a test substance, and
(2) a step of selecting a test substance that has bound to α₂δ in step (1) as a candidate for a prophylactic or therapeutic agent for tauopathy (hereinafter sometimes to be abbreviated as "the method of the present invention").

As shown in the Examples described below, the present inventors have clarified that gabapentine which is a ligand for an auxiliary subunit α₂δ that regulates the localization and the like of subunits constituting the calcium channel shows a remarkable cell death suppressive effect. It is considered that gabapentine binds to α₂δ to inhibit the functions thereof, as a result of which indirectly inhibits the intracellular influx of calcium ion by VDCC and exhibits prophylactic or therapeutic effects on tauopathy. Therefore, if a substance that binds to α₂δ can be obtained by screening, the substance can be used as a candidate for a prophylactic or therapeutic agent for tauopathy.

The α₂δ to be used in the method of the present invention may be any of α₂δ-1, α₂δ-2, α₂δ-3, and α₂δ-4, and α₂δ-1, α₂δ-2, and α₂δ-3 are preferred, and α₂δ-1 and α₂δ-2 are particularly preferred. It may be a part of these and, for example, all or a part of the α₂ domain, all or a part of each δ domain, and the like can be mentioned. Also, α₂δ may be in the form of a fusion protein of α₂ and δ such as the above-mentioned Pro-form, or α₂ and δ may be bonded by a disulfide bond or the like. When provided in the form of a fusion protein, α₂ and δ may be fused via a linker such as a GS linker.

As the above-mentioned α₂δ, those produced by a genetic engineering method or a chemical synthesis method may be used. Using a genetic engineering method, it can be produced, for example, by producing a nucleic acid encoding α₂δ based on known sequence information, and synthesizing a protein from the nucleic acid by using cells or in a cell-free system.

In the present invention, "binding" (also referred to as "bindability") between α₂δ and a test substance means that the both substances are so close to each other that an interaction can occur between them or a state of association thereof can be produced if they are brought into contact with each other. Examples of such interaction include a covalent bond, a coordination bond, an ionic bond, a hydrogen bond, a van der Waals bond, a hydrophobic interaction, and the like.

In addition, the presence or absence of the bindability between α₂δ and a test substance can be determined by measuring the index of binding activity or affinity. Examples of the index of the binding affinity include dissociation constant (K_{D}), and a binding constant (also referred to as an association constant) which is the reciprocal of the dissociation constant, and the like. The dissociation constant can be measured by a method known per se and, for example, a method using surface plasmon resonance, an isothermal titration calorimetry method, and the like can be mentioned.

Examples of the system for measuring and calculating surface plasmon resonance include, but are not limited to, a Biacore system (GE Healthcare), and the like. When using the Biacore system, for example, the K_{D} can be measured by the following procedure. (1) α₂δ is immobilized on the sensor chip of the Biacore system by amine coupling or the like, (2) α₂δ is brought into contact with a test substance in a solution containing the test substance prepared at a plurality of concentrations, (3) the interaction between them is detected by surface plasmon resonance, (4) a series of binding and dissociation reactions are drawn as a sensorgram with the horizontal axis as time and the vertical axis as the binding amount (RU), (5) the sensorgram created at respective concentrations is fitted into a 1:1 Langmuir model by using software (e.g., BIA evaluation software (GE Healthcare), etc.), and various velocity parameters are calculated, (6) the dissociation constant is calculated from various velocity parameters.

Examples of the system to be used for the isothermal titration calorimetry method include, but are not limited to, a MicroCal·system (GE Healthcare), and the like. When using the MicroCal system, for example, the K_{D} can be measured by the following procedure. (1) A solution containing a test substance is added dropwise and stirred in a sample cell containing α₂δ kept at a constant temperature, (2) heat is generated or absorbed in direct proportion to the amount of binding due to intermolecular interaction, and the temperature of the solution in the sample cell changes, (3) the cell feedback network (CFB) senses the temperature difference (ΔT) from the reference cell, (4) the reference cell or sample cell is heated so that ΔT becomes 0, (5) the feedback power required to maintain ΔT=0 is measured to obtain the amount of heat generated or absorbed by the interaction, and (6) the dissociation constant is calculated from the binding isotherm with the vertical axis as the developed amount of heat and the horizontal axis as the molar ratio of α₂δ and the test substance.

In the present specification, when measured using surface plasmon resonance, a K_{D} of not more than 1500 nM can be evaluated to show bindability to α₂δ, where lower values of K_{D} can be evaluated to show higher bindability to α₂δ. The K_{D} is preferably not more than 1000 nM, more preferably not more than 500 nM, further preferably not more than 300 nM, particularly preferably not more than 100 nM.

Examples of the test substance to be used in the method of the present invention include cell extracts, cell culture supernatants, microbial fermentation products, marine organism-derived extracts, plant extracts, purified proteins or crude proteins, peptides, non-peptide compounds, synthetic low-molecular-weight compounds, and natural compounds. The aforementioned test substance can also be obtained using any of the many approaches in combinatorial library methods known in the art, including (1) biological library, (2) synthetic library method using deconvolution, (3) "one-bead one-compound" library method, and (4) synthetic library method using affinity chromatography sorting. The biological library method using affinity chromatography sorting is limited to peptide library, but the other four approaches are applicable to libraries of peptides, non-peptide oligomers, or low-molecular-weight compounds (Lam (1997) Anticancer Drug Des. 12:145-67). Examples of the methods for synthesizing molecular libraries can be found in the art (DeWitt et al. (1993) Proc. Natl. Acad. Sci. USA 90:6909-13; Erb et al. (1994) Proc. Natl. Acad. Sci. USA 91:11422-6; Zuckermann et al. (1994) J. Med. Chem. 37:2678-85; Cho et al. (1993) Science 261:1303-5; Carell et al. (1994) Angew. Chem. Int. Ed. Engl. 33:2059; Carell et al. (1994) Angew. Chem. Int. Ed. Engl. 33:2061; Gallop et al. (1994) J. Med. Chem. 37:1233-51). The compound library can be made as solutions (Houghten (1992) Bio/Techniques 13:412-21) or as beads (Lam (1991) Nature 354:82-4), chips (Fodor (1993) Nature 364:555-6), bacteria (US Patent No. 5,223,409), spores (US Patent Nos. 5,571,698, 5,403,484, and 5,223,409), plasmids (Cull et al. (1992) Proc. Natl. Acad. Sci. USA 89:1865-9) or phages (Scott and Smith (1990) Science 249:386-90; Devlin (1990) Science 249:404-6; Cwirla et al. (1990) Proc. Natl. Acad. Sci. USA 87:6378-82; Felici (1991) J. Mol. Biol. 222:301-10; US-A-2002-103360).

A test substance (hereinafter to be also referred to as "candidate substance") selected as a candidate for the prophylactic or therapeutic agent for tauopathy by the method of the present invention may be further evaluated for an inhibitory effect on misfolding of tau protein in neurons. The inhibitory effect may be evaluated as an effect of inhibiting the intracellular accumulation or extracellular release of misfolded tau protein. Such evaluation method may be a method including, for example, (1) a step of adding a candidate substance to a neuron associated with a pathology of tauopathy, and detecting or measuring the intracellular amount or the amount contained in the medium (amount in the medium) of misfolded tau protein and (2) a step of evaluating a candidate compound whose aforementioned intracellular amount or medium amount is lower than that before addition of the candidate substance or a control group free of addition as a compound that inhibits the intracellular accumulation or extracellular release of misfolded tau protein. As used herein, a low value refers to a value of 90%, 80%, 70%, 60%, 50%, or 40% or less with respect to the intracellular amount or the medium amount (control value) of the control group.

The "misfolded tau protein" in the present invention is preferably a "tau oligomer". The "tau oligomer" refers to an aggregate in which 3 to 50 tau (monomers) are associated, and may be either a soluble tau oligomer or an insoluble tau oligomer. The soluble tau oligomer may be, for example, an oligomer that is dissolved in 1% Polyoxyethylene(10) Octylphenyl Ether (Triton X-100, CAS No. 9002-93-1) and then recovered in the supernatant by a centrifugation operation at about 10,000xg. The insoluble tau oligomer may be an oligomer that is recovered as a precipitate after the centrifugation operation. The tau oligomer of the present invention does not include tau fibers (e.g., PHF, etc.) which are fibrous structures in which insoluble tau oligomers are bound to each other.

The tau oligomer in the present invention may undergo various chemical modifications including phosphorylation. It may also form a complex with other proteins and/or nucleic acids (DNA, RNA).

The amount of misfolded tau protein can be measured by a method well known to those skilled in the art (e.g., Patent Literature 2, Patent Literature 3). Examples thereof include, but are not limited to, dot blot analysis using anti-tau protein antibody and a Western blotting method and an ELISA method using antibodies specific for misfolded tau proteins (e.g., TOC1 antibody, antibody described in WO 2011/026031, and the like) .

The neuron associated with the tauopathy pathology and used in the above-mentioned evaluation method may be a neuron induced to differentiate from pluripotent stem cells (iPS cells) established from somatic cells collected from a tauopathy patient. The iPS cells can be appropriately produced by a method known per se using somatic cells collected from a tauopathy patient. As a method for inducing differentiation of iPS cells into neurons, various known differentiation-inducing methods can be appropriately selected and used. For example, a method of forcibly expressing Ngn2 (e.g., human Ngn2 protein: NP_076924, mouse Ngn2 protein: NP_033848) in cells to differentiate them into cerebral cortical neurons can be mentioned. The tauopathy patient is not particularly limited, but a patient with Alzheimer's disease or FTLD-tau as described above is preferred, and further, a FTLD-Tau patient having a mutation in the MAPT gene (e.g., FTDP-17 patient) is particularly desirable.

The present invention is described in more detail in the following by referring to Examples which are mere examples and do not limit the present invention in any way.

### [Example]

### Example 1: Effect in tauopathy cell model

### [Materials and methods]

### Ethics statement

The use of human iPS cells was approved by the Ethics Committee of the Graduate School of Medicine and Faculty of Medicine, Kyoto University. All methods were performed according to approved guidelines. Formal informed consent was obtained from all test subjects.

### Production of iPS cell-derived cerebral cortical neurons by induction of Ngn2 expression

Using two types of iPS cell lines (FTLD-Tau1 and FTLD-Tau2) established by the method described in Non Patent Literature 1, the following experiment was performed. FTLD-Tau1 is a stable iPS cell line obtained by introducing the Ngn2 gene-which is regulated by a tetracycline-inducible promoter-into an iPS cell line established from the cells of an FTLD-Tau patient having an intron mutation (intron 10 + 14C→T) in MAPT. FTLD-Tau2 is a stable iPS cell line obtained by introducing the aforementioned Ngn2 gene into an iPS cell line established from the cells of an FTLD-Tau patient having an exon mutation (R406W) in MAPT. These iPS cell lines were dissociated into single cells using actase and seeded together with 1 µg/ml doxicycline (Clontech) on a matrigel-coated plastic plate or coverslip, by using a neuronal medium having a 1:1 ratio of DMEM/F12 (Life Technologies) and containing Neurobasal (Life Technologies), 1% N2 supplement, 2% B27 supplement, 10 ng/ml brain-derived neurotrophic factor (BDNF, R&D Systems), 10 ng/ml glial cell-derived neurotrophic factor (GDNF, R&D Systems) and 10 ng/ml neurotrophin-3 (NT-3; R&D Systems). In this method, not less than 90% of the cells generally differentiate into cerebral cortical neurons 7 days after the start of culture in a medium containing doxycycline. In addition, most of the cerebral cortical neurons obtained by this method are considered to be glutamatergic (Patent Literature 1). In the Examples of the present application, cerebral cortical neurons induced to differentiate from FTLD-Tau1 or FTLD-Tau2 by the aforementioned method is sometimes referred to as tauopathy neurons.

In the Examples of the present application, the day when the culture was started in a medium containing doxycycline (= the day when differentiation induction was started) is taken as Day0, and the number of days from the start date is sometimes indicated by "Day"+"number".

### Immunocyte chemistry

The cells were fixed with 4% paraformaldehyde at room temperature for 30 min, washed with PBS, and permeabilized with PBS containing 0.2% Triton X-100 for 10 min at room temperature. Then, the cells were blocked with Block Ace (Yukijirushi) for 30 min. After incubation with the primary antibody at 4°C overnight, the cells were washed 3 times with PBS, and incubated with the appropriate secondary antibody for 1 hr at room temperature. Cell images were taken from Delta Vision (Applied Precision) or IN Cell Analyzer 6000 (GE Healthcare). Cell numbers were quantified with IN Cell Analyzer 6000 and IN CELL Developer toolbox software 1.9 (GE Healthcare). The following primary antibodies were used in this assay: βIII tubulin (1:2,000, Covance), NeuN (1:500, Millipore). The staining results are shown in Fig. 1. As shown in Fig. 1, the cell body and neurite of a neuron become βIII tubulin-positive (green on the image), and the nucleus in the aforementioned cell body was NeuN-positive (it becomes yellow to orange because it overlaps with cell body color (green) on the image). Therefore, in the Examples of the present application, the number of viable βIII tubulin-positive cell bodies or the number of βIII tubulin-NeuN double-positive dots was measured as the number of viable neurons (number of tubulin-positive neuronal cell bodies).

### Westernblot analysis

Cells were harvested and lysed in RIPA buffer containing 0.1% SDS, 150 mM NaCl, 1% NP-40, 0.5% deoxycholate, 50 mM Tris-HCl (pH 8.0), protease inhibitor (Roche), and phosphatase inhibitor (Roche). After sonication, the sample was centrifuged at 13,000 × g for 15 min at 4°C and the supernatant was recovered. The concentration of the proteins in the supernatant was measured using a bicinchoninic acid (BCA) assay kit (Pierce), SDS-PAGE (20 µg per lane) was performed under non-reducing conditions, and Westernblot was performed using TOC1 antibody that specifically recognizes misfolded tau protein. Signal intensity was analyzed using ImageQuant LAS 4000 (GE Healthcare). The tau antibodies used in this paper are shown in Table 3.

**[Table 1]**

| antibody | target | host species | supplier |
|---|---|---|---|
| Tau12 | human tau | Ms (IgG) | Millipore |
| TOC1 | oligomer tau | Ms (IgM) | Kanaan/Binder Lab |

### Dot blot analysis

### <Preparation of medium supernatant>

The medium was recovered from the cell culture system and subjected to low-speed centrifugation (1,000 rpm, 3 min) to precipitate cell debris, and the obtained supernatant was recovered as the culture supernatant. Each culture supernatant (500 µl) was transferred to a tube with an ultrafiltration filter (Vivaspin, cutoff molecular weight: 10 kD, GE Healthcare) and concentrated to 50 µl. Each concentrated sample (2 µl) was blotted on a nitrocellulose membrane.

### <Preparation of cell extract>

The cells were harvested from the aforementioned cell culture system, suspended in TBS (Tris buffered saline) containing a protease inhibitor and a phosphatase inhibitor, and then subjected to sonication to disrupt the cells. The cell homogenate was centrifuged (13,000 × g, 15 min), and the supernatant was recovered as a cell extract. Each cell extract was blotted on a nitrocellulose membrane (hole diameter 0.45 µm, GE Healthcare) at 1.2 µg protein/spot.

The nitrocellulose membrane obtained by the above-mentioned method was subjected to various tau antibody treatments, detection (Western Lightning Plus-ECL, PerkinElmer), and signal quantification (ImageQuant LAS4000, GE Healthcare) according to conventional methods. Tau12 was used as a human tau-specific antibody, and TOC1 antibody was used as a tau oligomer-specific antibody. In the Example of the present application, a TOC1 antibody (Non Patent Literature 1) distributed by Dr. Nicholas Kanaan of the Department of Translational Science and Molecular Medicine, College of Human Medicine, Michigan State University (USA) was used.

### Cell survival assay

A test compound was added to the medium of FTLD-Tau1 or FTLD-Tau2 on Day8, the cells were fixed on Day21 and immunostained with βIII tubulin (cell density at seeding on Day0 was 5×10⁴ cells/well). As a control, cells of Day8 without addition of the test compound were fixed, cryopreserved, and then immunostained at the same time as the aforementioned cells of Day21. The number of βIII tubulin-positive cells (= number of viable neurons) was measured with IN Cell Analyzer 6000 (GE Healthcare), and the value obtained by the following formula was calculated as the cell survival rate.

In the assay using pregabalin and mirogabalin, the test compound was added to the medium of FTLD-Tau2 on Day10, and the cells were fixed on Day21 and the above-mentioned analysis was performed.

### Analysis of tau oligomer formation

The cells of Day21 obtained by the above-mentioned cell survival assay were subjected to the aforementioned Westernblot analysis and dot blot analysis to analyze the amount of tau oligomer.

### [numerical formula 1]

cell survival rate (%) = (number of βIII tubulin-positive cells on Day21/number of βIII tubulin-positive cells on Day8) × 100

### Statistical analysis

The results were analyzed using one-way ANOVA followed by Tukey post hoc analysis or Student's t-test in order to determine statistical significance. The difference was taken as significant at p<0.05. The analysis was performed using SPSS software (IBM). All bar graphs show mean±SEM.

### Therapeutic drug for epilepsy

The action effects of the 6 kinds of therapeutic drugs for epilepsy used in the Example are shown below.

**[Table 2]**

| action | | Gabapentine | Topiramate | Carbamazepine | Levetiracetam | Valproic Acid | Clonazepam | Reference |
|---|---|---|---|---|---|---|---|---|
| Na channel inhibition | | | ○ | ○ | | ○ | | 1 |
| voltage-dependent Ca channel inhibition | 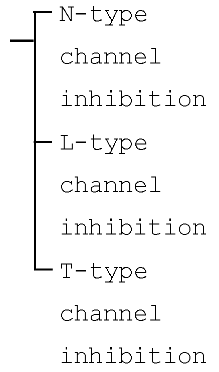 | | | | ○ | | | 2 |
| | | | ○ | | | | | 3 |
| | | | | | | ○ | | 4 |
| binding to α2δ subunit of voltage-dependent Ca channel | | ○ | | | | | | 5 |
| mitochondrial Na/Ca exchange transporter inhibition | | | | | | | ○ | 6 |

Of the above, clonazepam is best known as an agonist of the benzodiazepine receptor in the postsynaptic membrane of GABAergic neurons, but most of the cerebral cortical neurons obtained by the aforementioned method are glutamatergic (Patent Literature 1). Therefore, the present specification describes the action as an inhibitor of mitochondrial Na/Ca exchange transporter. The documents in the Table are as follows.
Reference 1: GABAPEN Tablets Pharmaceutical Interview Form Revised April 2017 (11th Edition)
Reference 2: Topina Tablets Pharmaceutical Interview Form Revised April 2017
Reference 3: Carbamazepine Tablets Pharmaceutical Interview Form Revised June 2018 (5th Edition)
Reference 4: E Keppra Tablets Pharmaceutical Interview Form Revised June 2018 (17th edition)
Reference 5: Depakene Tablets Pharmaceutical Interview Form Revised July 2019
Reference 6: Elinor J. Griffiths et al., Inhibition of mitochondrial calcium efflux by clonazepam in intact single rat cardiomyocytes and effects on NADH production. Cell Calcium, 21(4): 321-329, 1997.

### [Results]

About 200 kinds of existing drugs and 6 kinds of existing drugs known as therapeutic drugs for epilepsy (Gabapentine, Topiramate, Carbamazepine, Levetiracetam, Valproic Acid, and Clonazepam) were analyzed for cell death suppressive effects on cerebral cortical neurons (tauopathy neurons) derived from tauopathy patients, by using the above-mentioned method.

As a result, in the analysis of about 200 kinds of existing drugs, no drug showed a very remarkable cell death suppressive effect (results not disclosed).

Furthermore, among the 6 kinds of therapeutic drugs for epilepsy, the existing drugs known as inhibitors of voltage-dependent calcium channel L type (Topiramate), N type (Levetiracetam), and T type (Valproic Acid) were analyzed up to 50 µM. However, a significant cell death suppressive effect was not observed (Fig. 2). In addition, when treated with clonazepam, which inhibits the Na/Ca exchange transporter (mitochondrial Na/Ca exchanger) responsible for calcium release from mitochondria and is also used for the treatment of manic depression, or carbamazepine, which is an inhibitor of sodium channel, a cell death suppressive effect was not observed (Fig. 2) .

In contrast, a very remarkable cell death suppressive effect was surprisingly observed with gabapentine. As shown in Fig. 2, gabapentin significantly suppressed spontaneous cell death of tauopathy neurons in a wide range of 12-50 µM.

Gabapentine is a ligand that binds with high affinity to the α₂δ subunit which is one of the auxiliary subunits of voltage-dependent calcium channel. The α₂δ subunit is not a constituent component of the ion channel, but is responsible for the transport of the α₁ subunit that constitutes the channel to the plasma membrane, and the like, and it has been reported that the transport is inhibited by the binding of gabapentine, but the voltage-dependent calcium channel current is hardly inhibited directly (Hendrich J et al., Pharmacological disruption of calcium channel trafficking by the alpha2delta ligand gabapentin. Proc Natl AcadSci U S A. 105:3628-33, 2008.).

Therefore, from the above-mentioned results, it was clarified clear that, surprisingly, the cell death of tauopathy neurons is effectively suppressed by the ligand of α₂δ, which is an auxiliary subunit of the channel, rather than the existing drugs that can directly inhibit the voltage-dependent calcium channel.

Then, it was examined whether the above-mentioned suppression of cell death by gabapentine was accompanied by the suppression of the formation of tau oligomers. The verification was performed by the above-mentioned Western blot analysis and dot blot analysis. The results are shown in Fig. 3. In both the Westernblot analysis and the dot blot analysis, the tau oligomer levels significantly decreased in the cells cultured in the presence of gabapentine (even by 10 µM gabapentine treatment) as compared to cells without gabapentine treatment. Furthermore, it was shown by the dot blot analysis that, in gabapentine-treated cells, both the amount of tau oligomer in the culture supernatant and the amount of tau oligomer in the cells significantly decreased.

Therefore, it was shown that gabapentine significantly inhibits misfolding of tau protein and formation of tau oligomer that occur in tauopathy neurons.

Subsequently, the above-mentioned analysis was also performed on other compounds known as inhibitors of α₂δ. Since gabapentine enacarbil is a prodrug of gabapentine, pregabalin and mirogabalin were analyzed. The results of the cell survival assay are shown in Fig. 4, and the results of Westernblot analysis and dot blot analysis are shown in Fig. 5.

Pregabalin and mirogabalin very remarkably suppressed spontaneous cell death of tauopathy neurons in a wide range of 6-50 µM (Fig. 4). In the cells treated with these medicaments, both the amount of tau oligomer in the culture supernatant and the amount of intracellular tau oligomer decreased very remarkably (Fig. 5). In particular, the effect on the amount of intracellular tau oligomer was dramatic, and it was clarified that treatment with pregabalin or mirogabalin can reduce the amount of tau oligomer accumulated in the cell by about 84 - 90%.

Therefore, it was shown that not only gabapentine but also pregabalin and mirogabalin can significantly inhibit the misfolding of tau protein and the formation of tau oligomer that occur in tauopathy neurons, and can effectively suppress cell death.

From the above, it was shown that an agent that binds to the α₂δ subunit-which is an auxiliary subunit of voltage-dependent calcium channel-inhibits oligomerization of tau protein, effectively suppresses cell death of cerebral cortical neurons, and can be a prophylactic or therapeutic agent for tauopathy.

### Example 2: Effect in tauopathy animal model

Subsequently, the effect of the inhibitor of α₂δ in vivo was analyzed. Specifically, gabapentine was orally administered to a tauopathy mouse model, and a suppressive effect on intracerebral tau aggregation was analyzed.

### [Materials and methods]

### Mouse model

As a tauopathy mouse model, rTg4510 mice (Santacruz, K., et al., Science, 309:476-481, 2005) that overexpress human P301L mutant tau in the cerebral cortex and hippocampal region were used. This mouse is known to show tau lesions (structural abnormalities and aggregation of tau) in the cerebral cortex and hippocampal region from 2-3 months of age, and progressive brain atrophy with nerve loss at 5-6 months of age.

### Drug administration

40 mg/kg gabapentine was orally administered to about 4-month-old rTg4510 mice 4 times a week for 6 weeks (for administration concentration, see Reda, H.M., et al., Eur J Pharmacol, 771:162-172, 2016). Gabapentine (100 mg) was dissolved in 2.5 ml of ultrapure water, diluted 10-fold with drinking water immediately before administration, and orally given in an amount of body weight (g) × 10 µl (gabapentine administration group; 3 mice). As the non-administration control, rTg4510 mouse of the same age was orally administered with the same amount of drinking water on the same schedule and used (control; one mouse).

In the tauopathy mouse model, tau lesions progress rapidly in a short period of 2-3 months. In order to ensure the accuracy of the analysis results, mice of exactly the same age in days were used. Also, the gender was unified to male.

### Evaluation of tau pathology by positron emission tomography (PET)

Radioactive ligand [18F]PM-PBB3 was injected under anesthesia from the tail vein into mice (about 5.5 months of age) after completion of oral administration, and imaging was performed using Focus 220 PET scanner (Siemens) (Tagai et al., 2020, MedRxiv). Tagai et al., 2020, MedRxiv). PM-PBB3 is known to selectively bind to various tau aggregates that accompany tauopathy, from tau fibers with further elongation of tau oligomers to neurofibrillary change with further aggregation of tau fibers. Therefore, by injecting [¹⁸F]PM-PBB3 into a living body and detecting the radioactivity using PET, the formation and accumulation of tau structure aggregates can be detected with high sensitivity.

### Brain volume measurement by nuclear magnetic resonance imaging (MRI)

T2-weighted MRI was performed using a 7T horizontal MRI scanner (Brucker), ROIs (region of interests) were determined from the captured images, and the volumes of the cerebral cortex, hippocampus, and cerebellum were calculated.

### [Results]

Of the three gabapentine-administered mice, one had a body weight of about 9% less than the average body weight of the other two mice at the start of administration, and was excluded from the analysis because the body weight did not increase thereafter. As a result, the body weight range (at the start of administration) of all the mice used in this analysis was very narrow and was -2.1% to +2.9%, thus providing an analysis system more suitable for drug efficacy evaluation.

The results of time series measurement of [¹⁸F]radioactivity (time-radioactivity curve) in the hippocampus, cerebral cortex, and cerebellum from immediately after intravenous injection of radioactive ligand to 60 min after the intravenous injection in the gabapentine-administered mice and the non-administered control mouse are shown in Fig. 7A. Radioactivity was detected immediately after intravenous injection in any site of any mouse, and then rapidly attenuated, indicating that the time course is almost the same between the administered and non-administered mice. Although the results are omitted, it was confirmed that there was no significant difference in the volumes of the hippocampus, cerebral cortex, and cerebellum between the administered and non-administered mice in this analysis.

Standardized uptake value ratio (SUVR) images of a cross section of the brain including the cerebral cortex and hippocampus 40 - 60 min after intravenous injection of radioactive ligand are shown in Fig. 7B. The upper images are SUVR alone, and in the lower images, an SUVR image and an image by nuclear magnetic resonance imaging (MRI) are superimposed, where each SUVR image was calculated with the cerebellum as the target area. It is clear that [¹⁸F]radioactivity in the hippocampus and cerebral cortex is lower (blackish on the image) in the gabapentine-administered mice than in the non-administered mice.

A graph of the mean values obtained in Fig. 7B after 40 - 60 min is shown in Fig. 7C. It is clear that the [¹⁸F]radioactivity was lower in both the hippocampus and the cerebral cortex in the gabapentine-administered mice than in the non-administered mice.

These results reveal that gabapentine-administered mice had less accumulation of tau aggregates in the cerebral cortex and hippocampus than non-administered mice. Therefore, it was clarified that oral ingestion of gabapentine can effectively suppress tau lesions that occur in the brain of the tauopathy mouse model.

From the foregoing, it was clarified that an agent that binds to the α₂δ subunit which is an auxiliary subunit of voltage-dependent calcium channel can effectively suppress tau lesions that occur in the living brain, and can be used as a prophylactic or therapeutic agent for tauopathy.

### [Industrial Applicability]

An inhibitor of α₂δ of the voltage-dependent calcium channel is useful for the prophylaxis and/or treatment of tauopathy, and a screening method using the bindability to α₂δ as an index is useful for screening for a prophylaxis and/or therapeutic agent for tauopathy.

This application is based on a patent application No. 2019-194758 filed in Japan (filing date: October 25, 2019), the contents of which are entirely incorporated in full herein by reference.

## Claims

1. A prophylactic or therapeutic agent for tauopathy, comprising an inhibitor of α₂δ of a voltage-dependent calcium channel.

2. The agent according to claim 1, wherein the inhibitor of α₂δ is a compound represented by the formula (1): 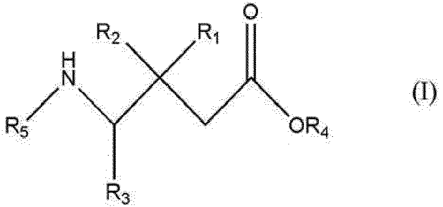 [in the formula (I),
R₁ is a straight chain or branched alkyl group having 1 - 6 carbon atoms;
R₂ is a hydrogen atom or a straight chain or branched alkyl group having 1 - 6 carbon atoms, or R₁ and R₂ are bonded to form a cycloalkane having 4 - 6 carbon atoms and optionally condensed with a 5-membered carbocycle, wherein the 5-membered carbocycle is optionally substituted by an alkyl group having 1 - 6 carbon atoms, an alkenyl group having 2 - 6 carbon atoms or a cycloalkyl group having 3 - 7 carbon atoms;
R₃ is a hydrogen atom, a methyl group or a carboxyl group;
R₄ is a hydrogen atom or a straight chain or branched alkyl group having 1 - 6 carbon atoms;
R₅ is a hydrogen atom or a group represented by the formula (II): 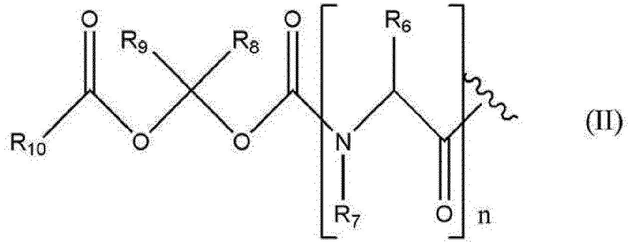 [in the formula (II),
n is 0 or 1;
R₆ is hydrogen, alkyl, substituted alkyl, alkoxy, substituted alkoxy, acyl, substituted acyl, alkoxycarbonyl, substituted alkoxycarbonyl, aryl, substituted aryl, arylalkyl, substituted arylalkyl, carbamoyl, substituted carbamoyl, cycloalkyl, substituted cycloalkyl, cycloheteroalkyl, substituted cycloheteroalkyl, heteroalkyl, substituted heteroalkyl, heteroaryl, substituted heteroaryl, heteroarylalkyl or substituted heteroarylalkyl, or R₆ and R₇ optionally form, together with an atom bonded thereto, cycloheteroalkyl or a substituted cycloheteroalkyl ring;
R₇ is hydrogen, alkyl, substituted alkyl, aryl, substituted aryl, arylalkyl, substituted arylalkyl, cycloalkyl, substituted cycloalkyl, cycloheteroalkyl, substituted cycloheteroalkyl, heteroalkyl, substituted heteroalkyl, heteroaryl, substituted heteroaryl, heteroarylalkyl or substituted heteroarylalkyl;
R₈ and R₉ are each independently hydrogen, alkyl, substituted alkyl, alkoxycarbonyl, substituted alkoxycarbonyl, aryl, substituted aryl, arylalkyl, substituted arylalkyl, carbamoyl, substituted carbamoyl, cycloalkyl, substituted cycloalkyl, heteroalkyl, substituted heteroalkyl, heteroaryl, substituted heteroaryl, heteroarylalkyl or substituted heteroarylalkyl, or R₈ and R₉ optionally form, together with an atom bonded thereto, cycloalkyl, substituted cycloalkyl, cycloheteroalkyl or a substituted cycloheteroalkyl ring; and
R₁₀ is acyl, substituted acyl, alkyl, substituted alkyl, aryl, substituted aryl, arylalkyl, substituted arylalkyl, cycloalkyl, substituted cycloalkyl, cycloheteroalkyl, substituted cycloheteroalkyl, heteroalkyl, substituted heteroalkyl, heteroaryl, substituted heteroaryl, heteroarylalkyl or substituted heteroarylalkyl]],
or a salt thereof.

3. The agent according to claim 2, wherein the inhibitor of α₂δ is one or more compounds selected from gabapentine, pregabalin, mirogabalin and gabapentine enacarbil, and analogs thereof, or a salt thereof.

4. The agent according to claim 3, wherein the inhibitor of α₂δ is one or more compounds selected from gabapentine and pregabalin, or a salt thereof.

5. The agent according to any one of claims 1 to 4, wherein the agent is an inhibitor of misfolding of a tau protein.

6. The agent according to any one of claims 1 to 4, wherein the tauopathy is Alzheimer's disease or frontotemporal lobar degeneration showing tauopathy (FTLD-Tau).

7. The agent according to claim 6, wherein the tauopathy is caused by MAPT gene mutation.

8. A method for screening for a prophylactic or therapeutic agent for tauopathy, comprising the following steps:
(1) a step of contacting α₂δ of a voltage-dependent calcium channel with a test substance, and
(2) a step of selecting a test substance that has bound to α₂δ in step (1) as a candidate for a prophylactic or therapeutic agent for tauopathy.

9. A method for preventing or treating tauopathy in a mammal, comprising administering an effective amount of an inhibitor of α₂δ to the mammal.

10. The method according to claim 9, wherein the inhibitor of α₂δ is a compound represented by the formula (1): 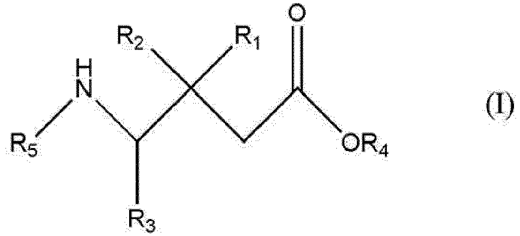 [in the formula (I),
R₁ is a straight chain or branched alkyl group having 1 - 6 carbon atoms;
R₂ is a hydrogen atom or a straight chain or branched alkyl group having 1 - 6 carbon atoms, or R₁ and R₂ are bonded to form a cycloalkane having 4 - 6 carbon atoms and optionally condensed with a 5-membered carbocycle, wherein the 5-membered carbocycle is optionally substituted by an alkyl group having 1 - 6 carbon atoms, an alkenyl group having 2 - 6 carbon atoms or a cycloalkyl group having 3 - 7 carbon atoms;
R₃ is a hydrogen atom, a methyl group or a carboxyl group;
R₄ is a hydrogen atom or a straight chain or branched alkyl group having 1 - 6 carbon atoms; and
R₅ is a hydrogen atom or a group represented by the formula (II): 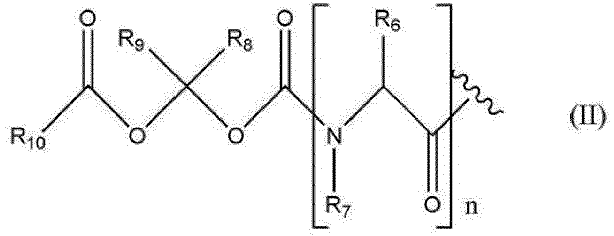 [in the formula (II),
n is 0 or 1;
R₆ is hydrogen, alkyl, substituted alkyl, alkoxy, substituted alkoxy, acyl, substituted acyl, alkoxycarbonyl, substituted alkoxycarbonyl, aryl, substituted aryl, arylalkyl, substituted arylalkyl, carbamoyl, substituted carbamoyl, cycloalkyl, substituted cycloalkyl, cycloheteroalkyl, substituted cycloheteroalkyl, heteroalkyl, substituted heteroalkyl, heteroaryl, substituted heteroaryl, heteroarylalkyl or substituted heteroarylalkyl, or R₆ and R₇ optionally form, together with an atom bonded thereto, cycloheteroalkyl or a substituted cycloheteroalkyl ring;
R₇ is hydrogen, alkyl, substituted alkyl, aryl, substituted aryl, arylalkyl, substituted arylalkyl, cycloalkyl, substituted cycloalkyl, cycloheteroalkyl, substituted cycloheteroalkyl, heteroalkyl, substituted heteroalkyl, heteroaryl, substituted heteroaryl, heteroarylalkyl or substituted heteroarylalkyl;
R₈ and R₉ are each independently hydrogen, alkyl, substituted alkyl, alkoxycarbonyl, substituted alkoxycarbonyl, aryl, substituted aryl, arylalkyl, substituted arylalkyl, carbamoyl, substituted carbamoyl, cycloalkyl, substituted cycloalkyl, heteroalkyl, substituted heteroalkyl, heteroaryl, substituted heteroaryl, heteroarylalkyl or substituted heteroarylalkyl, or R₈ and R₉ optionally form, together with an atom bonded thereto, cycloalkyl, substituted cycloalkyl, cycloheteroalkyl or a substituted cycloheteroalkyl ring; and
R₁₀ is acyl, substituted acyl, alkyl, substituted alkyl, aryl, substituted aryl, arylalkyl, substituted arylalkyl, cycloalkyl, substituted cycloalkyl, cycloheteroalkyl, substituted cycloheteroalkyl, heteroalkyl, substituted heteroalkyl, heteroaryl, substituted heteroaryl, heteroarylalkyl or substituted heteroarylalkyl]],
or a salt thereof.

11. The method according to claim 9 or 10, wherein the inhibitor of α₂δ is one or more compounds selected from gabapentine, pregabalin, mirogabalin and gabapentine enacarbil, and analogs thereof, or a salt thereof.

12. An inhibitor of α₂δ for use in the treatment or prophylaxis of tauopathy.

13. The inhibitor of α₂δ according to claim 12, wherein the inhibitor is a compound represented by the formula (1): 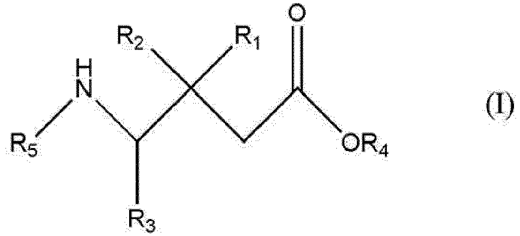 [in the formula (I),
R₁ is a straight chain or branched alkyl group having 1 - 6 carbon atoms;
R₂ is a hydrogen atom or a straight chain or branched alkyl group having 1 - 6 carbon atoms, or R₁ and R₂ are bonded to form a cycloalkane having 4 - 6 carbon atoms and optionally condensed with a 5-membered carbocycle, wherein the 5-membered carbocycle is optionally substituted by an alkyl group having 1 - 6 carbon atoms, an alkenyl group having 2 - 6 carbon atoms or a cycloalkyl group having 3 - 7 carbon atoms;
R₃ is a hydrogen atom, a methyl group or a carboxyl group;
R₄ is a hydrogen atom or a straight chain or branched alkyl group having 1 - 6 carbon atoms; and
R₅ is a hydrogen atom or a group represented by the formula (II): 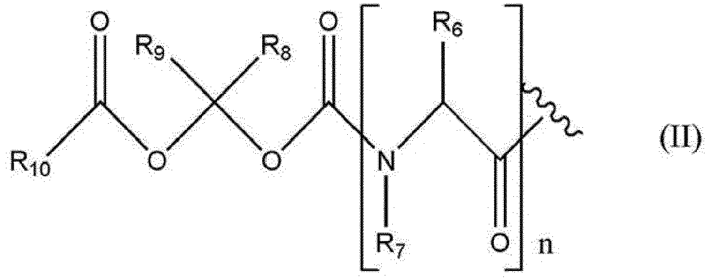 [in the formula (II),
n is 0 or 1;
R₆ is hydrogen, alkyl, substituted alkyl, alkoxy, substituted alkoxy, acyl, substituted acyl, alkoxycarbonyl, substituted alkoxycarbonyl, aryl, substituted aryl, arylalkyl, substituted arylalkyl, carbamoyl, substituted carbamoyl, cycloalkyl, substituted cycloalkyl, cycloheteroalkyl, substituted cycloheteroalkyl, heteroalkyl, substituted heteroalkyl, heteroaryl, substituted heteroaryl, heteroarylalkyl or substituted heteroarylalkyl, or R₆ and R₇ optionally form, together with an atom bonded thereto, cycloheteroalkyl or a substituted cycloheteroalkyl ring;
R₇ is hydrogen, alkyl, substituted alkyl, aryl, substituted aryl, arylalkyl, substituted arylalkyl, cycloalkyl, substituted cycloalkyl, cycloheteroalkyl, substituted cycloheteroalkyl, heteroalkyl, substituted heteroalkyl, heteroaryl, substituted heteroaryl, heteroarylalkyl or substituted heteroarylalkyl;
R₈ and R₉ are each independently hydrogen, alkyl, substituted alkyl, alkoxycarbonyl, substituted alkoxycarbonyl, aryl, substituted aryl, arylalkyl, substituted arylalkyl, carbamoyl, substituted carbamoyl, cycloalkyl, substituted cycloalkyl, heteroalkyl, substituted heteroalkyl, heteroaryl, substituted heteroaryl, heteroarylalkyl or substituted heteroarylalkyl, or R₈ and R₉ optionally form, together with an atom bonded thereto, cycloalkyl, substituted cycloalkyl, cycloheteroalkyl or a substituted cycloheteroalkyl ring; and
R₁₀ is acyl, substituted acyl, alkyl, substituted alkyl, aryl, substituted aryl, arylalkyl, substituted arylalkyl, cycloalkyl, substituted cycloalkyl, cycloheteroalkyl, substituted cycloheteroalkyl, heteroalkyl, substituted heteroalkyl, heteroaryl, substituted heteroaryl, heteroarylalkyl or substituted heteroarylalkyl]],
or a salt thereof.

14. The inhibitor of α₂δ according to claim 12 or 13, wherein the inhibitor is one or more compounds selected from gabapentine, pregabalin, mirogabalin and gabapentine enacarbil, and analogs thereof, or a salt thereof.
